# EUROPÄISCHE PATENTSCHRIFT

(11) **EP 3 802 842 B1**
(45) Veröffentlichungstag und Bekanntmachung des Hinweises auf die Patenterteilung: **13.04.2022**
(21) Anmeldenummer: 19728686.7
(22) Anmeldetag: 05.06.2019
(51) Int. Cl.: C12P 13/04

(54) **VERFAHREN ZUR HERSTELLUNG VON AMINOBENZOESÄURE ODER EINES AMINOBENZOSÄURE-FOLGEPRODUKTS**
METHOD FOR THE PREPARATION OF AMINOBENZOIC ACID OR AN AMINOBENZOIC ACID DERIVATIVE
PROCÉDÉ DE PRODUCTION D'ACIDE AMINOBENZOÏQUE OU D'UN PRODUIT DÉRIVÉ D'ACIDES AMINOBENZOÏQUES

(30) Priorität: 07.06.2018 EP 18176433
(43) Veröffentlichungstag der Anmeldung: 14.04.2021
(73) Patentinhaber: Covestro Intellectual Property GmbH & Co. KG, 51373 Leverkusen (DE)
(72) Erfinder: KLOECKNER, Wolf, 51065 Köln (DE); BEGGEL, Franz, 50935 Köln (DE); JÄGER, Gernot, 50733 Köln (DE); KLAFFL, Simon, 40597 Düsseldorf (DE); OLF, Guenter, 53909 Zülpich (DE)
(74) Vertreter: Levpat
(86) Internationale Anmeldenummer: PCT/EP2019/064627
(87) Internationale Veröffentlichungsnummer: WO 2019/234092

(56) Entgegenhaltungen:
- EP-A1- 2 562 263
- WO-A1-2015/124687
- DE-A1- 10 149 869
- QIAN-ZHU LI ET AL: "Recovery processes of organic acids from fermentation broths in the biomass-based industry", JOURNAL OF MICROBIOLOGY AND BIOTECHNOLOGY,, Bd. 26, 25. September 2015 (2015-09-25), Seiten 1-8, XP002762999, in der Anmeldung erwähnt

## Beschreibung

Die vorliegende Erfindung betrifft ein Verfahren zur Herstellung von Aminobenzoesäure oder eines Aminobenzoesäure-Folgeprodukts unter Anwendung eines Fermentationsprozesses, bei welchem
(I) die in der durch die Fermentation erhaltene Fermentationsbrühe gebildete Aminobenzoesäure teilweise, gegebenenfalls soweit aufgrund des Löslichkeitsgleichgewichtes möglich, als unlösliches Calcium-Aminobenzoat gebunden, dieses unlösliche Calcium-Aminobenzoat anschließend
(II) entweder als solches oder im Gemisch mit den in der Fermentation verwendeten Mikroorganismen isoliert und unter Abscheidung eines von Calcium-Aminobenzoat verschiedenen unlöslichen Calcium-Salzes in eine wasserlösliche Form überführt und dann
(III) durch Einleiten von Kohlenstoffdioxid unter Druck in die vom ausgefallenen Calcium-Salz befreite wässrige Lösung Aminobenzoesäure ausgefällt wird.

Die Herstellung organischer Säuren durch fermentative Prozesse hat in der jüngeren Vergangenheit besondere Aufmerksamkeit erfahren. Dabei ist insbesondere die Isolierung des gewünschten Produkts aus der erhaltenen Fermentationsbrühe in einer wirtschaftlichen Art und Weise nicht trivial. Die Isolierung von organischen Säuren aus Fermentationsbrühen war bereits Gegenstand wissenschaftlicher Untersuchungen; siehe beispielsweise J. Microbiol. Biotechnol. (2016), 26(1), 1-8. Dort wird unter anderem die Gewinnung organischer Säuren wie Milchsäure, Zitronensäure und Bernsteinsäure durch Ausfällung als Ammonium- oder Calcium-Salze beschrieben. Beispielhaft wird die Isolierung durch Ausfällung mit Ca(OH)₂ oder CaCO₃ gefolgt von Filtration und Auflösen der abfiltrieren Calcium-Salze mit Schwefelsäure beschrieben. Der Verbrauch an Calcium-Salzen und die Bildung wenig wertvollen Calcium-Sulfats werden als Nachteile dieser Methode beschrieben. Mit der Gewinnung solcher und anderer organischer Säuren aus fermentativer Herstellung befassen sich auch weitere Schriften; siehe EP 2 562 263 A1 und Chem. Biochem. Eng. Q. 19 (2) 159-172 (2005) (Milchsäure), US 2010/0094051 A1 (Bernsteinsäure) sowie Chemical Engineering and Processing, 81 (2014) 59-71 (Glutaminsäure).

Die deutsche Patentanmeldung DE 101 49 869 A1 betrifft ein Verfahren zur Isolierung von Salzen von organischen Säuren aus einer wässrigen Lösung, insbesondere aus einem Fermentationsaustrag, durch partielle Verdampfungskristallisation und anschließender oder gleichzeitiger Verdrängungsfällung ihres Salzes sowie zur Freisetzung der organischen Säure aus dem Kristallisat, vorzugsweise mittels eines Elektromembranverfahrens. In einer bevorzugten Ausführungsform ist die organische Säure eine Carbonsäure, insbesondere Polyhydroxycarbonsäuren wie 2-Keto-polyhydroxy-C6-carbonsäuren. Weiter werden Ketogulonsäuren, Milchsäure, Zitronensäure, Vanillinsäure, Idonsäure und Gulonsäure als mögliche Säuren genannt, wobei die Ketogulonsäuren 2,4-Diketo-D-gulonsäure, 2,5-Diketo-D-gulonsäure, 2-Keto-L-gulonsäure und Ascorbinsäure besonders hervorgehoben werden.

Unter den organischen Säuren, die fermentativ zugänglich sind, ist auch Aminobenzoesäure als ein wirtschaftlich bedeutsames Produkt hervorzuheben, das entweder als solches oder als Zwischenprodukt in der Herstellung anderer, von der Aminobenzoesäure durch weitere chemische Umsetzung(en) abgeleiteter Verbindungen (fortan als *Aminobenzoesäure-Folgeprodukte* bezeichnet), Anwendung findet. Aminobenzoesäure findet bspw. Anwendung in der Herstellung von Farbstoffen, Geruchsstoffen oder Pharmazeutika (Wiklund, Current Organic Synthesis, 2006, 3, 379-402). Ein Beispiel für eine wichtige Folgereaktion von Aminobenzoesäure zur Herstellung eines anderen Produkts ist die Decarboxylierung zu Anilin, das seinerseits insbesondere als Intermediat in der Herstellung von Isocyanaten von Bedeutung ist.

Auch Aminobenzoesäure hat das Interesse wissenschaftlicher Untersuchungen gefunden. So untersuchten Decker und Frye bereits 1966 die Eigenschaften des Substitutionsmusters auf die Chelat-bildenden Eigenschaften von Aminobenzoesäuren (vgl. Z. Naturforschg. 21 b, 522 - 526 [1966]). Chelate von Aminobenzoat mit Mg(II), Ca(II), Cr(III), Mn(II), Fe(II), Fe(III), Co(II), Ni(II), Cu(II), Cd(II) und Zn(II) wurden untersucht.

Die fermentative Herstellung von Aminobenzoesäure ist in der Literatur beschrieben. Beispielhaft sei auf Balderas-Hemandez, V. E. et al., "Metabolic engineering for improving anthranilate synthesis from glucose in Escherichia coli", Microb. Cell. Fact. 2009, 8, 19 (doi: 10.118611475-2859-8-19) verwiesen. Auch in der Patentliteratur finden sich hierzu Veröffentlichungen; siehe beispielsweise Anmeldungen WO 2015/124686 A1 und WO 2015/124687 A1 sowie die darin jeweils zitierte Literatur. Fermentationsprozesse laufen in einer wässrigen Umgebung ab und liefern im Falle der Herstellung von Aminobenzoesäure wässrige Produktgemische *(Fermentationsbrühen)* mit einem Massengehalt an Aminobenzoesäure im Bereich von 10,0 g/L bis 100 g/L.

Besondere Bedeutung hat das ortho-Isomer der Aminobenzoesäure, die *Anthranilsäure.* Im Stoffwechsel von Bakterien und Hefen wird Anthranilsäure im Shikiminsäureweg als natürliches Intermediat bei der Synthese von Tryptophan gebildet. Bei der biotechnologischen Produktion von Anthranilsäure wird deren Umsetzung im Stoffwechselweg reduziert oder unterbunden, um eine Akkumulation im Fermentationsmedium zu erzielen. Ein solches Konzept zur biotechnologischen Produktion von Anthranilsäure und ihrer anschließenden katalytischen Umsetzung zu Anilin ist in den bereits erwähnten internationalen Patentanmeldungen WO 2015/124686 A1 und WO 2015/124687 A1 beschrieben. Als möglicher rekombinanter Mikroorganismus wird die Verwendung von Bakterien aus der Familie der Corynebakterien oder Pseudomonaden beschrieben. Eine jüngere Anmeldung (WO 2017/102853 A1) beschreibt den Einsatz von Hefen.

Ist die fermentative Produktion von Anthranilsäure in einem neutralen bis alkalischen pH-Bereich angestrebt (wie WO 2015/124686 A1 und WO 2015/124687 A1 beschrieben), so ist die Zugabe einer Base erforderlich, um die Säure zu neutralisieren und eine zu starke pH-Reduktion bei der Fermentation zu vermeiden. Anthranilsäure liegt in einem neutralen bis alkalischen pH-Bereich gelöst als Anion *(Aminobenzoat)* vor. Welches Kation in der Fermentationsbrühe vorliegt, hängt von der zur Neutralisation verwendeten Base ab. In einer Ausführungsform der beschriebenen Verfahren wird im Anschluss an die Fermentation der pH-Wert durch Zugabe einer Säure auf einen Wert von oder nahebei pH 3,5 (isoelektrischer Punkt) reduziert, gelöstes Aminobenzoat also wieder in die Säure bzw. das "Zwitterion" ^{_}OOC-C₆H₄-NH₃⁺) überführt. Aufgrund der geringen Restlöslichkeit von Aminobenzoesäure fällt diese dabei als kristalliner Feststoff aus und kann so isoliert werden. Dieses Verfahren hat zwei wesentliche Nachteile, die im Folgenden beschrieben werden:
1. Durch Zugabe einer Base zur Vermeidung einer pH-Reduktion in der Fermentation und durch Zugabe eine Säure zur pH-Reduktion in der anschließenden Kristallisation wird im Produktionsverfahren kontinuierlich Base und Säure verbraucht, was mit offensichtlichen wirtschaftlichen Nachteilen verbunden ist. Das bei der Kristallisation entstehende Salz liegt in der Mutterlauge gelöst vor und muss entsprechen entsorgt werden.
2. Das in der Fermentation gelöst vorliegende Aminobenzoat kann bei zu großen Massenanteilen in der Fermentationsbrühe inhibierend auf die Stoffwechselaktivität der Bakterien wirken, wodurch die weitere Produktion von Aminobenoesäure reduziert wird oder im Extremfall sogar zum Erliegen kommt.

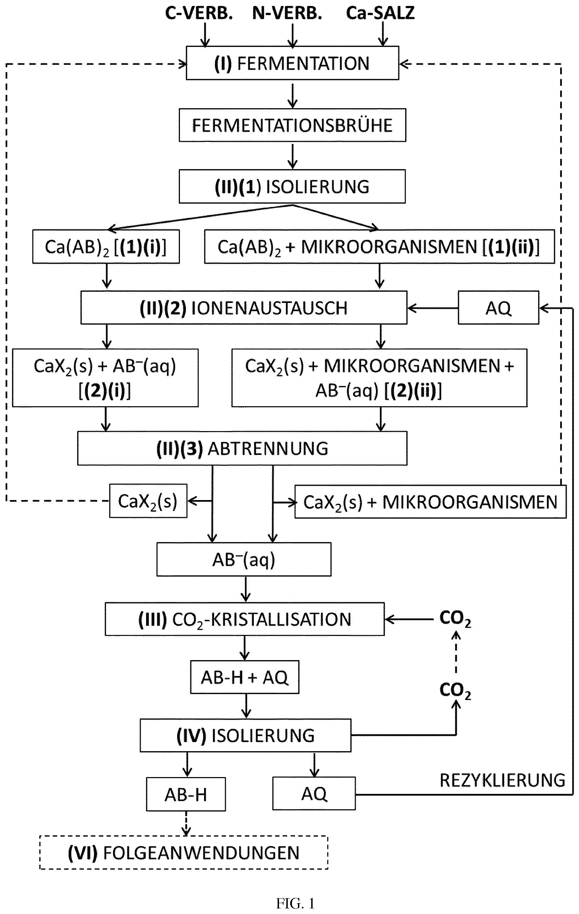

Es bestand daher nach wie vor ein Bedarf an weiteren Verbesserungen auf dem Gebiet der fermentativen Produktion von Aminobenzoesäure. Vollkommen überraschend wurde gefunden, dass die oben genannten Probleme gelöst oder zumindest in ihren Auswirkungen reduziert werden können, wenn (**I**) die in der Fermentationsbrühe gebildete Aminobenzoesäure (die überwiegend bis vollständig als Anion, *Aminobenzoat,* vorliegt) teilweise, gegebenenfalls soweit aufgrund des Löslichkeitsgleichgewichtes möglich, als unlösliches Calcium-Aminobenzoat gebunden, dieses unlösliche Calcium-Aminobenzoat anschließend (**II**) entweder als solches oder im Gemisch mit Mikroorganismen isoliert und unter Abscheidung eines von Calcium-Aminobenzoat verschiedenen unlöslichen Calcium-Salzes in eine wasserlösliche Form überführt und dann **(III)** durch Einleiten von Kohlenstoffdioxid unter Druck in die vom ausgefallenen Calcium-Salz befreite wässrige Lösung Aminobenzoesäure ausgefällt wird. Dies ist im beigefügten Blockdiagramm (vgl. **FIG. 1****)** schematisch dargestellt. Optionale Schritte sind dort durch gestrichelte Pfeile bzw. Blöcke gekennzeichnet. Im Folgenden verwendete fettgedruckte Bezeichnungen in Klammern verweisen auf dieses Diagramm. Ein Gegenstand der vorliegenden Erfindung ist demnach ein **Verfahren zur Herstellung von Aminobenzoesäure oder eines Aminobenzoesäure-Folgeprodukts,** umfassend die folgenden Schritte:
(I) Fermentieren eines Rohstoffes, der wenigstens
   - eine fermentierbare Kohlenstoff-haltige Verbindung **(C-VERB.),** bevorzugt ausgewählt aus Stärkehydrolysat, Zuckerrohrsaft, Zuckerrübensaft, Hydrolysaten aus lignocellulosehaltigen Rohstoffen oder Mischungen derselben (d. h. Mischungen aus zwei oder mehr der vorgenannten Verbindungen),
   und
   - eine Stickstoff-haltige Verbindung **(N-VERB.)**, bevorzugt ausgewählt aus Ammoniakgas, Ammoniakwasser, Ammoniumsalzen (insbesondere anorganische Ammoniumsalze wie Ammoniumchlorid und/oder Ammoniumsulfat, bevorzugt Ammoniumsulfat), Harnstoff oder Mischungen derselben (d. h. Mischungen aus zwei oder mehr der vorgenannten Verbindungen),
   umfasst,
   in einem Fermentationsreaktor unter Verwendung von Mikroorganismen und eines anorganischen Calcium-Salzes **(Ca-SALZ),**
   sodass ein in einer wässrigen Fermentationslösung suspendiertes Gemisch umfassend nicht gelöste Mikroorganismen und ausgefallenes Calcium-Aminobenzoat **(FERMENTATIONSBRÜHE)** erhalten wird **(FERMENTATION);**
(**II)**
   (1) Isolieren des in Schritt **(I)** angefallenen
      (1)(i) Calcium-Aminobenzoats **(Ca(AB)₂)**
      oder
      (1)(ii) Gemisches umfassend nicht gelöste Mikroorganismen **(MIKROORGANISMEN)** und ausgefallenes Calcium-Aminobenzoat **(Ca(AB)₂)**
      aus der wässrigen Fermentationslösung - **(ISOLIERUNG),**
   (2) Überführen des im Calcium-Aminobenzoat gebundenen Aminobenzoats in eine wasserlösliche Form unter Bildung eines von Calcium-Aminobenzoat verschiedenen wasserunlöslichen Calcium-Salzes durch Zugabe einer wässrigen Phase **(AQ)**, die wasserlösliche Aminobenzoat-Salze bildende Kationen und wasserunlösliche Calcium-Salze bildende Anionen enthält, zu dem isolierten Calcium-Aminobenzoat aus (1)(i) oder zu dem isolierten Gemisch umfassend nicht gelöste Mikroorganismen und ausgefallenes Calcium-Aminobenzoat aus (1)(ii), wobei die zugegebene wässrige Phase **(AQ)**
      Lithium-, Natrium-, Kalium- und/oder Ammonium-Kationen, bevorzugt Ammonium-Kationen,
      und
      Carbonat und/oder Hydrogencarbonat-Anionen
      umfasst,
      sodass eine Suspension enthaltend
         (2)(i) das ausgefallene wasserunlösliche Calcium-Salz **(CaX₂** (s)) oder
         (2)(ii) ein Gemisch umfassend nicht gelöste Mikroorganismen **(MIKROORGANISMEN)** und das wasserunlösliche Calcium-Salz **(CaX₂ (s))**
      in einer wässrigen Lösung von Aminobenzoat **(AB⁻(aq))** erhalten wird **(IONENAUSTAUSCH),**
   (3) Abtrennen der in Schritt (2) angefallenen wässrigen Lösung von Aminobenzoat **(AB⁻(aq))** von dem ausgefallenen wasserunlöslichen Calcium-Salz aus (2)(i) oder von dem Gemisch umfassend nicht gelöste Mikroorganismen und das wasserunlösliche Calcium-Salz aus (2)(ii), bevorzugt gefolgt von einer Rückführung des wasserunlöslichen Calcium-Salzes aus (2)(i) oder des Gemisches umfassend nicht gelöste Mikroorganismen und das wasserunlösliche Calcium-Salz aus (2)(ii) in Schritt (I) (ABTRENNUNG);
(III) Einleiten von Kohlenstoffdioxid **(CO₂)** bei einem Druck größer oder gleich 1,50 bar_{(abs)}, bevorzugt bei einem Druck im Bereich von 5,00 bar_{(abs}.₎ bis 100 bar_{(abs)}, besonders bevorzugt bei einem Druck im Bereich von 7,00 bar_{(abs}.₎ bis 40,0 bar_{(abs)}, ganz besonders bevorzugt bei einem Druck im Bereich von 20,0 bar_{(abs)} bis 30,0 bar_{(abs)}, in die in Schritt **(II)**(3) abgetrennte wässrige Lösung von Aminobenzoat unter Abscheidung von Aminobenzoesäure, sodass eine Suspension **(AB-H + AQ)** enthaltend Aminobenzoesäure **(AB-H)** in einer wässrigen Lösung **(AQ)** gebildet wird **(CO₂-KRISTALLISATION);**
(IV) Isolierung der in Schritt (III) abgeschiedenen Aminobenzoesäure **(AB-H)** umfassend eine Druckerniedrigung unter Freisetzung von Kohlenstoffdioxid (CO₂), wobei eine an Kohlenstoffdioxid abgereicherte und von abgeschiedener Aminobenzoesäure befreite wässrige Lösung **(AQ)** erhalten wird **(ISOLIERUNG);**
(V) Verwenden der in Schritt (IV) erhaltenen, an Kohlenstoffdioxid abgereicherten und von abgeschiedener Aminobenzoesäure befreiten wässrigen Lösung **(AQ)** als Bestandteil der in Schritt (II)(2) zugegebenen wässrigen Phase **(REZYKLIERUNG);**
(VI) optionale weitere Umsetzung der in Schritt (**IV)** isolierten Aminobenzoesäure **(AB-H)** zu einem Aminobenzoesäure-Folgeprodukt, wobei Schritt (VI) eine der folgenden Umsetzungen umfasst:
   (1) Decarboxylierung der Aminobenzoesäure zu Anilin;
   (2) Decarboxylierung der Aminobenzoesäure zu Anilin, gefolgt von säurekatalysierter Reaktion des Anilins mit Formaldehyd unter Bildung von Di- und Polyaminen der Diphenylmethanreihe;
   (3) Decarboxylierung der Aminobenzoesäure zu Anilin, gefolgt von säurekatalysierter Reaktion des Anilins mit Formaldehyd unter Bildung von Di- und Polyaminen der Diphenylmethanreihe, gefolgt von Umsetzung mit Phosgen unter Bildung von Di- und Polyisocyanaten der Diphenylmethanreihe;
   (4) Decarboxylierung der Aminobenzoesäure zu Anilin, gefolgt von Umsetzung des Anilins zu einer Azoverbindung;
   (5) Umsetzung der Aminobenzoesäure zu einem Amid;
   (6) Umsetzung der Aminobenzoesäure zu leitenden Polymeren wie insbesondere Polyanthranilsäure -

### (FOLGEANWENDUNGEN).

Der Begriff *"Aminobenzoesäure-Folgeprodukt"* bezeichnet im Rahmen der vorliegenden Erfindung ein Produkt, das durch weitere chemische Umwandlung von Aminobenzoesäure erhalten wird.

Die *Fermentation* findet in einem wässrigen Medium statt und liefert ein Produktgemisch enthaltend feste Bestandteile, die in einer wässrigen Lösung aufgeschlämmt (suspendiert) sind. Dieses Produktgemisch in seiner Gesamtheit wird im Rahmen der vorliegenden Erfindung im Einklang mit der in der Fachliteratur üblichen Nomenklatur als *Fermentationsbrühe* bezeichnet. Die Fermentationsbrühe ist also ein zweiphasiges Gemisch aus in einer wässrigen Lösung und aufgeschlämmten (suspendierten) unlöslichen Feststoffen. Die suspendierten Feststoffe enthalten die eingesetzten Mikroorganismen (oder zumindest einen Teil davon) und ausgefallenes Calcium-Aminobenzoat. Die wässrige Lösung (also die Fermentationsbrühe ohne die darin suspendierten festen Bestandteile) wird im Rahmen der vorliegenden Erfindung als *wässrige Fen-nentations**lösung* bezeichnet.

Unter einer *fermentierbaren Kohlenstoff-haltigen Verbindung* wird jede organische Verbindung oder Mischung organischer Verbindungen verstanden, die von den rekombinanten Zellen des eingesetzten Mikroorganismus verwendet werden kann, um Aminobenzoesäure (bzw., abhängig vom pH-Wert, Aminobenzoat) zu produzieren.

Der Begriff *wasserunlöslich* oder *unlöslich* ist im Rahmen der vorliegenden Erfindung selbstverständlich nicht in einem absoluten Sinne zu verstehen (da es so etwas wie eine absolute Unlöslichkeit auch gar nicht gibt). So ist mit dem Ausdruck "*Überführen des im Calcium-Aminobenzoat gebundenen Aminobenzoats in eine wasserlösliche Form unter Bildung eines von Calcium-Aminobenzoat verschiedenen wasserunlöslichen Calcium-Salzes durch Zugabe einer wässrigen Phase, die wasserlösliche Aminobenzoat-Salze bildende Kationen und wasserunlösliche Calcium-Salze bildende Anionen enthält"* in Schritt (II)(2) selbstverständlich nicht gemeint, dass ausnahmslos alle Calcium-Ionen als Salz des zugegebenen Anions ausgefällt werden. Gemeint ist vielmehr, dass die zugegebenen Anionen ein Calcium-Salz bilden, dessen Löslichkeitsprodukt hinreichend klein ist, um unter den gegebenen Randbedingungen überschritten zu werden, und dass das Löslichkeitsprodukt des Aminobenzoat-Salzes der eingesetzten Kationen hinreichend groß ist, um unter den gegebenen Randbedingungen *nicht* überschritten zu werden. Daher kann Aminobenzoat in Lösung gehen und dadurch "freigewordene" Calcium-Ionen entsprechend als Salze des zugegebenen Anions ausfallen.

Im Rahmen der vorliegenden Erfindung bezeichnen *pH-Werte* den bei 20 °C gemessenen pH-Wert, sofern nichts anderes angegeben ist.

Folgende **Abbildungen** sind zum besseren Verständnis der Erfindung beigefügt:
- **FIG. 1**: zeigt ein Blockdiagramm des erfindungsgemäßen Verfahrens. Schritte, die in der breitesten Ausgestaltung der Erfindung optional sind, sind gestrichelt dargestellt.

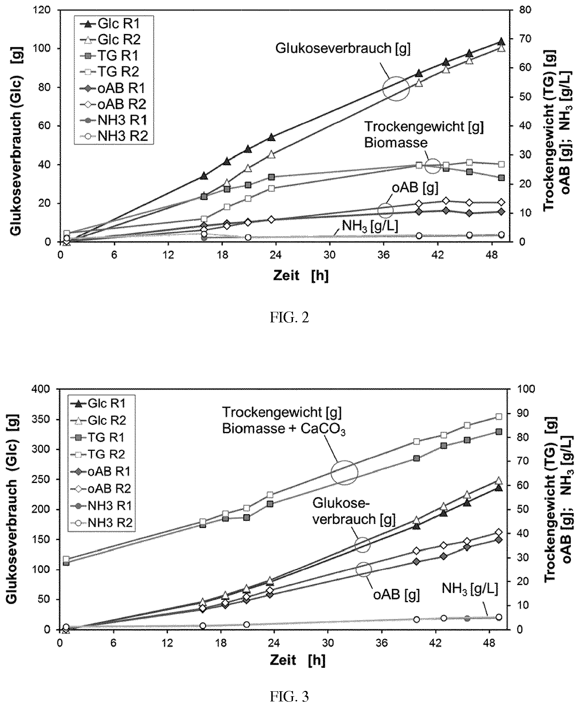

- **FIG. 2**: zeigt eine Fed-batch Fermentation eines ortho-Aminobenzoat produzierenden Stammes von *C. glutamicum* gemäß dem Stand der Technik. Der Versuch wurde unter ansonsten gleichen Bedingungen in zwei Fermentationsreaktoren parallel durchgeführt. Ausgefüllte Symbole kennzeichnen Reaktor 1, offene Symbole kennzeichnen Reaktor 2.
- **FIG. 3**: zeigt eine Fed-batch Fermentation eines ortho-Aminobenzoat produzierenden Stammes von *C. glutamicum* in Gegenwart von CaCO₃ gemäß Schritt (I) der vorliegenden Erfindung. Der Versuch wurde unter ansonsten gleichen Bedingungen in zwei Fermentationsreaktoren parallel durchgeführt. Ausgefüllte Symbole kennzeichnen Reaktor 1, offene Symbole kennzeichnen Reaktor 2.

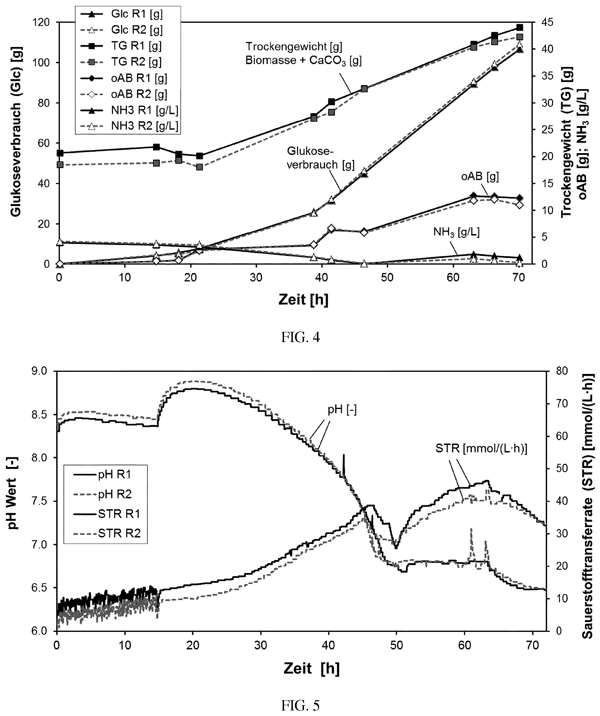

- **FIG. 4 und FIG. 5**: zeigen die Ergebnisse einer Fed-batch Fermentation eines ortho-Aminobenzoat produzierenden Stammes von C. *glutamicum* in Gegenwart von CaCO₃ gemäß Schritt (I) der vorliegenden Erfindung. Der Versuch wurde unter ansonsten gleichen Bedingungen in zwei Fermentationsreaktoren parallel durchgeführt. Ausgefüllte Symbole kennzeichnen Reaktor 1, offene Symbole kennzeichnen Reaktor 2.

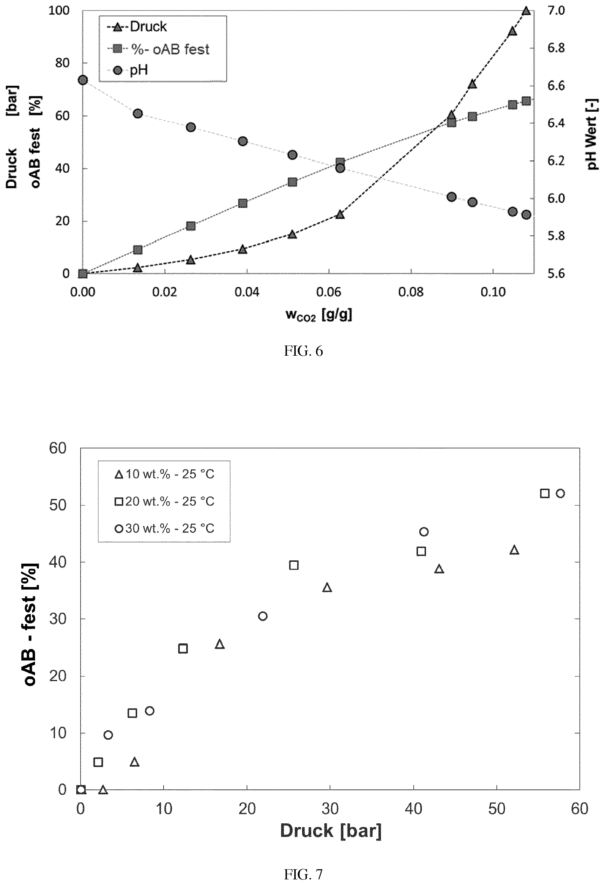

- **FIG. 6**: zeigt die Ergebnisse einer Simulation der Kristallisation von ortho-Aminobenzoesäure mit CO₂ in einem abgeschlossenen Behälter. Auf der Primär-Achse sind der Druck in bar sowie der ausgefallene Anteil an Anthranilsäure in % (n_{oAB},_{fest}/n_{oAB},ₜₒₜₐₗ x 100) über dem Massenanteil von CO₂ (w_{co2}) in der flüssigen Phase aufgetragen. Die Sekundärachse zeigt den Verlauf des pH-Wertes. Die Symbole kennzeichnen Punkte aus der Simulation; die gestichelten Linien wurden zur verbesserten Visualisierung hinzugefügt.
- **FIG. 7**: zeigt die experimentellen Ergebnisse einer Kristallisation von ortho-Aminobenzoesäure mit CO₂ in einem abgeschlossenen Behälter. Auf der Primär-Achse ist der ausgefallene Anteil an Anthranilsäure in % (n_{oAB,fest}/n_{oAB,total} x 100) über dem Druck an CO₂ (bar) aufgetragen. Die Anfangskonzentrationen der NH₄-ortho-Aminobenzoat-Lösungen betrugen 10, 20 und 30 Massen-%.

Es folgt zunächst eine Kurzzusammenfassung verschiedener möglicher Ausführungsformen.

In einer **ersten Ausführungsform** der Erfindung, die mit allen anderen Ausführungsformen kombiniert werden kann, wird das in Schritt (I) eingesetzte anorganische Calcium-Salz ausgewählt aus Calciumcarbonat, Calciumhydrogencarbonat, Calciumhydroxid, Calciumoxid oder Mischungen derselben (d. h. Mischungen aus zwei oder mehr der vorgenannten Calcium-Salze), wobei bevorzugt eine Mischung aus Calciumcarbonat und Calciumhydroxid als Calcium-Salz eingesetzt wird.

In einer **zweiten Ausführungsform** der Erfindung, die mit allen anderen Ausführungsformen, sofern diese nicht eine kontinuierliche Ausgestaltung der Fermentation zum Gegenstand haben, kombiniert werden kann, wird die Fermentation in Schritt (I) diskontinuierlich in Fermentationszyklen durchgeführt.

In einer **dritten Ausführungsform** der Erfindung, die eine besondere Ausgestaltung der zweiten Ausführungsform ist, wird nach Abschluss eines Fermentationszyklus
- Schritt (II)(1) durchgeführt, indem die in Schritt (I) erhaltene wässrige Fermentationslösung unter Zurückhaltung des in dieser suspendierten Gemisches umfassend nicht gelöste Mikroorganismen und ausgefallenes Calcium-Aminobenzoat aus dem Fermentationsreaktor ausgeschleust wird;
- Schritt (II)(2) durchgeführt, indem die wässrige Phase in den Fermentationsreaktor gegeben wird, sodass im Fermentationsreaktor eine Suspension enthaltend ein Gemisch umfassend nicht gelöste Mikroorganismen und das wasserunlösliche Calcium-Salz in einer wässrigen Lösung von Aminobenzoat erhalten wird;
und
- Schritt (II)(3) durchgeführt, indem die in Schritt (II)(2) erhaltene wässrige Lösung von Aminobenzoat aus dem Fermentationsreaktor ausgeschleust wird, wobei das Gemisch umfassend nicht gelöste Mikroorganismen und das wasserunlösliche Calcium-Salz zurückgehalten wird und für den nächsten Fermentationszyklus zur Verfügung steht.

In einer **vierten Ausführungsform** der Erfindung, die eine andere besondere Ausgestaltung der zweiten Ausführungsform ist, wird nach Abschluss eines Fermentationszyklus
- Schritt (II)(1) durchgeführt, indem die in Schritt (I) erhaltene wässrige Fermentationslösung gemeinsam mit dem in dieser suspendierten Gemisch umfassend nicht gelöste Mikroorganismen und ausgefallenes Calcium-Aminobenzoat aus dem Fermentationsreaktor ausgeschleust wird und das Gemisch umfassend nicht gelöste Mikroorganismen und ausgefallenes Calcium-Aminobenzoat außerhalb des Fermentationsreaktors von der wässrigen Fermentationslösung getrennt und in den Fermentationsreaktor zurückgeführt wird;
- Schritt (II)(2) durchgeführt, indem die wässrige Phase in den Fermentationsreaktor gegeben wird, sodass im Fermentationsreaktor eine Suspension enthaltend ein Gemisch umfassend nicht gelöste Mikroorganismen und das wasserunlösliche Calcium-Salz in einer wässrigen Lösung von Aminobenzoat erhalten wird;
und
- Schritt (II)(3) durchgeführt, indem die in Schritt (II)(2) erhaltene wässrige Lösung von Aminobenzoat aus dem Fermentationsreaktor ausgeschleust wird, wobei das Gemisch umfassend nicht gelöste Mikroorganismen und das wasserunlösliche Calcium-Salz zurückgehalten wird und für den nächsten Fermentationszyklus zur Verfügung steht.

In einer **fünften Ausführungsform** der Erfindung, die eine weitere besondere Ausgestaltung der zweiten Ausführungsform ist, wird nach Abschluss eines Fermentationszyklus
- Schritt (II)(1) durchgeführt, indem die in Schritt (I) erhaltene wässrige Fermentationslösung gemeinsam mit dem in dieser suspendierten Gemisch umfassend nicht gelöste Mikroorganismen und ausgefallenes Calcium-Aminobenzoat aus dem Fermentationsreaktor ausgeschleust wird und das Gemisch umfassend nicht gelöste Mikroorganismen und ausgefallenes Calcium-Aminobenzoat außerhalb des Fermentationsreaktors von der wässrigen Fermentationslösung getrennt und in einen vom Fermentationsreaktor verschiedenen Behälter gegeben wird;
- Schritt (II)(2) durchgeführt, indem die wässrige Phase in diesen Behälter gegeben wird, sodass in diesem Behälter eine Suspension enthaltend ein Gemisch umfassend nicht gelöste Mikroorganismen und das wasserunlösliche Calcium-Salz in einer wässrigen Lösung von Aminobenzoat erhalten wird;
und es wird
- nach der Abtrennung der in Schritt (II)(2) erhaltenen wässrigen Lösung von Aminobenzoat von dem Gemisch umfassend nicht gelöste Mikroorganismen und das wasserunlösliche Calcium-Salz in Schritt (II)(3) dieses abgetrennte Gemisch in einem Schritt (II)(4) wieder in den Fermentationsreaktor gegeben, womit es für den nächsten Fermentationszyklus zur Verfügung steht.

In einer **sechsten Ausführungsform** der Erfindung, die mit allen anderen Ausführungsform, welche die diskontinuierliche Fermentation in Fermentationszyklen *(Schritt (I)*) zum Gegenstand haben, kombiniert werden kann, werden die Schritte (I) und (II) wiederholt, bis in Schritt (IV) die gewünschte Menge an Aminobenzoesäure erhalten wird oder die in Schritt (I) eingesetzten Mikroorganismen erneuert werden müssen.

In einer **siebten Ausführungsform** der Erfindung, die mit allen anderen Ausführungsformen, sofern diese nicht eine diskontinuierliche Ausgestaltung der Fermentation zum Gegenstand haben, kombiniert werden kann, wird die Fermentation in Schritt (I) kontinuierlich durchgeführt.

In einer **achten Ausführungsform** der Erfindung, die eine besondere Ausgestaltung der siebten Ausführungsform ist, wird in der wässrigen Fermentationslösung suspendiertes Gemisch umfassend nicht gelöste Mikroorganismen und ausgefallenes Calcium-Aminobenzoat kontinuierlich aus dem Fermentationsreaktor ausgeschleust, und
- Schritt (II)(1) durchgeführt, indem nach erfolgter Ausschleusung die unlöslichen Mikroorganismen und ausgefallenes Calcium-Aminobenzoat voneinander und von der wässrigen Fermentationslösung getrennt werden;
- Schritt (II)(2) durchgeführt, indem die wässrige Phase zu dem so abgetrennten Calcium-Aminobenzoat gegeben wird;
wobei die in Schritt (II)(1) abgetrennten unlöslichen Mikroorganismen teilweise bis vollständig in den Fermentationsreaktor zurückgeführt werden.

In einer **neunten Ausführungsform** der Erfindung, die eine andere besondere Ausgestaltung der siebten Ausführungsform ist, wird in der wässrigen Fermentationslösung suspendiertes ausgefallenes Calcium-Aminobenzoat unter Zurückhaltung der nicht gelösten Mikroorganismen kontinuierlich aus dem Fermentationsreaktor ausgeschleust und
- Schritt (II)(1) durchgeführt, indem nach erfolgter Ausschleusung ausgefallenes Calcium-Aminobenzoat von der wässrigen Fermentationslösung getrennt wird;
- Schritt (II)(2) durchgeführt, indem die wässrige Phase zu dem so abgetrennten Calcium-Aminobenzoat gegeben wird.

In einer **zehnten Ausführungsform** der Erfindung, die eine weitere besondere Ausgestaltung der siebten Ausführungsform ist, wird in der wässrigen Fermentationslösung suspendiertes Gemisch umfassend nicht gelöste Mikroorganismen und ausgefallenes Calcium-Aminobenzoat kontinuierlich aus dem Fermentationsreaktor ausgeschleust und
- Schritt (II)(1) durchgeführt, indem nach erfolgter Ausschleusung das Gemisch umfassend nicht gelöste Mikroorganismen und ausgefallenes Calcium-Aminobenzoat von der wässrigen Fermentationslösung getrennt wird;
- Schritt (II)(2) durchgeführt, indem die wässrige Phase zu diesem so abgetrennten Gemisch gegeben wird;
und
- nach der Abtrennung der in Schritt (II)(2) erhaltenen wässrigen Lösung von Aminobenzoat von dem Gemisch umfassend nicht gelöste Mikroorganismen und das wasserunlösliche Calcium-Salz in Schritt (II)(3) dieses abgetrennte Gemisch in einem Schritt (II)(4) wieder in den Fermentationsreaktor gegeben, sodass es dort für die weitere kontinuierliche Fermentation zur Verfügung steht.

In einer **elften Ausführungsform** der Erfindung, die noch eine weitere besondere Ausgestaltung der siebten Ausführungsform ist, wird in der wässrigen Fermentationslösung suspendiertes Gemisch umfassend nicht gelöste Mikroorganismen und ausgefallenes Calcium-Aminobenzoat kontinuierlich aus dem Fermentationsreaktor ausgeschleust und
- Schritt (II)(1) durchgeführt, indem nach erfolgter Ausschleusung das Gemisch umfassend nicht gelöste Mikroorganismen und ausgefallenes Calcium-Aminobenzoat von der wässrigen Fermentationslösung getrennt wird;
- Schritt (II)(2) durchgeführt, indem die wässrige Phase zu diesem so abgetrennten Gemisch gegeben wird;
und
- nach der Abtrennung der in Schritt (II)(2) erhaltenen wässrigen Lösung von Aminobenzoat von dem Gemisch umfassend nicht gelöste Mikroorganismen und das wasserunlösliche Calcium-Salz in Schritt (II)(3) dieses abgetrennte Gemisch in einem Schritt (II)(4a) in die Bestandteile *nicht gelöste Mikroorganismen* und *wasserunlösliches Calcium-Salz* getrennt und in einem Schritt (II)(4b) einer der voneinander getrennten Bestandteile, bevorzugt das wasserunlösliche Calcium-Salz, wieder in den Fermentationsreaktor gegeben, wo es für die weitere kontinuierliche Fermentation zur Verfügung steht, wobei dem Fermentationsreaktor bevorzugt frische Mikroorganismen zugegeben werden.

In einer **zwölften Ausführungsform** der Erfindung, die mit allen anderen Ausführungsformen kombiniert werden kann, wird aus der in Schritt (II)(1) erhaltenen wässrigen Fermentationslösung durch Säurezugabe bis Erreichen eines pH-Werts im Bereich von 3,0 bis < 4,0 Aminobenzoesäure auskristallisiert, und die auskristallisierte Aminobenzoesäure wird isoliert, wobei eine an Aminobenzoesäure abgereicherte Mutterlauge verbleibt.

In einer **dreizehnten Ausführungsform** der Erfindung, die eine besondere Ausgestaltung der zwölften Ausführungsform ist, wird die an Aminobenzoesäure abgereicherte Mutterlauge durch eine Folge aus einem Adsorptionsschritt und einem Desorptionsschritt aufkonzentriert, wobei der Desorptionsschritt durch Elution mit einem Desorptionsmittel eines pH-Werts im Bereich von 6,0 bis 11,0 durchgeführt wird, wobei das so erhaltene Desorbat optional als weiterer Bestandteil, der in Schritt (II)(2) zugegebenen wässrigen Phase eingesetzt wird.

In einer **vierzehnten Ausführungsform** der Erfindung, die eine besondere Ausgestaltung der dreizehnten Ausführungsform ist, wird als Adsorptionsmittel im Adsorptionsschritt Aktivkohle eingesetzt.

In einer **fünfzehnten Ausführungsform** der Erfindung, die mit allen anderen Ausführungsformen, sofern diese nicht die Kristallisation von Aminobenzoesäure aus der in Schritt (II)(1) erhaltenen wässrigen Fermentationslösung durch Säurezugabe vorsehen, kombiniert werden kann, wird die Schritt (II)(1) erhaltene wässrige Fermentationslösung in die Fermentation aus Schritt (I) zurückgeführt.

In einer **sechzehnten Ausführungsform** der Erfindung, die mit allen anderen Ausführungsformen kombiniert werden kann, wird in Schritt (III) nur ein Teil, bevorzugt 5,0 % bis 90 %, des in der in Schritt (II)(3) erhaltenen wässrigen Lösung enthaltenen Aminobenzoats als Aminobenzoesäure abgeschieden.

In einer **siebzehnten Ausführungsform** der Erfindung, die mit allen anderen Ausführungsformen kombiniert werden kann, umfasst Schritt (IV) folgende Teilschritte:
(1) Trennen der abgeschiedenen Aminobenzoesäure und der wässrigen Lösung aus Schritt (III) bei einem Druck gleich dem oder größer als der Druck in Schritt (III),
(2) Entspannen der in Schritt (1) abgetrennten wässrigen Lösung unter Freisetzung von Kohlenstoffdioxid, wobei eine an Kohlenstoffdioxid abgereicherte wässrige Lösung erhalten wird.

In einer **achtzehnten Ausführungsform** der Erfindung, die mit allen anderen Ausführungsformen kombiniert werden kann, wird das in Schritt (II)(3) abgetrennte wasserunlösliche Calcium-Salz aus (2)(i) oder das Gemisch umfassend nicht gelöste Mikroorganismen und das wasserunlösliche Calcium-Salz aus (2)(ii) in Schritt (I) zurückgeführt.

In einer **neunzehnten Ausführungsform** der Erfindung, die mit allen anderen Ausführungsformen kombiniert werden kann, wird das in Schritt (IV) freigesetzte Kohlenstoffdioxid aufgefangen und in Schritt (III) eingesetzt.

In einer **zwanzigsten Ausführungsform** der Erfindung, die mit allen anderen Ausführungsformen kombiniert werden kann, werden in Schritt (I) Mikroorganismen einer Art umfassend *Escherichia coli, Pseudomonas putida, Corynebacterium glutamicum, Ashbya gossypii, Pichia pastoris, Hansenula polymorpha, Yarrowia lipolytica, Zygosaccharomyces bailii* oder *Saccharomyces cerevisiae* eingesetzt, wobei die eingesetzten Mikroorganismen bevorzugt nur aus Vertretern genau einer dieser Arten bestehen, wobei *Corynebacterium glutamicum* ATTC 13032 ganz besonders bevorzugt ist.

In einer **einundzwanzigsten Ausführungsform** der Erfindung, die mit allen anderen Ausführungsformen kombiniert werden kann, wird Schritt (I) bei einem pH-Wert von 4,0 oder größer, bevorzugt bei einem pH-Wert im Bereich von 4,0 bis 7,5, besonders bevorzugt im Bereich von 5,0 bis 7,0, durchgeführt.

In einer **zweiundzwanzigsten Ausführungsform** der Erfindung, die mit allen anderen Ausführungsformen, bevorzugt mit der zweiundzwanzigsten, kombiniert werden kann, wird Schritt (II)(2) bei einem pH-Wert von > 7,0, bevorzugt > 8,0, durchgeführt.

Die zuvor kurz geschilderten Ausführungsformen und weitere mögliche Ausgestaltungen der Erfindung werden im Folgenden näher erläutert. Dabei können die Ausführungsformen beliebig miteinander kombiniert werden, sofern sich aus dem Zusammenhang nichts anderes ergibt.

**Schritt (I)** des erfindungsgemäßen Verfahrens umfasst den eigentlichen **Fermentationsschritt,** während die späteren Schritte die Aufarbeitung und Weiterverarbeitung des in der Fermentation gebildeten Produktgemisches umfassen.

Aminobenzoesäure kommt in drei *isomeren Formen* (ortho-, meta- und para-Aminobenzoesäure) vor. Grundsätzlich kann das erfindungsgemäße Verfahren auf alle drei Isomere, entweder in isomerenreiner Form oder als Mischungen verschiedener Isomere, angewandt werden. Für alle Ausführungsformen der vorliegenden Erfindung gilt, dass die in Schritt (I) durch Fermentation herzustellende Aminobenzoesäure vorzugsweise das ortho-Isomer umfasst. Besonders bevorzugt umfasst die in Schritt (I) herzustellende Aminobenzoesäure mindestens 50,0 mol-%, ganz besonders bevorzugt mindestens 90,0 mol-%, bezogen auf die Gesamtstoffmenge aller vorhandenen Isomere an Aminobenzoesäure, des ortho-Isomers. Außerordentlich ganz besonders bevorzugt besteht die in Schritt (I) bereitzustellende Aminobenzoesäure aus dem ortho-Isomer in isomerenreiner (d. h. Isomerenreinheit > 99,0 mol-%) Form.

Die Fermentation in Schritt (I) wird bevorzugt so durchgeführt, dass der pH-Wert in der erhaltenen wässrigen Fermentationslösung einen Wert von 4,0 nicht unterschreitet, da bei geringeren pH-Werten - selbst wenn die Mikroorganismen dafür geeignet sind - Aminobenzoesäure nicht mehr hinreichend als Calcium-Salz gebunden wird. Der sich einstellende pH-Wert hängt dabei auch von der Art des eingesetzten Calcium-Salzes, genauer gesagt der Basizität des Gegenions ab. Bevorzugt wird Schritt (I) so durchgeführt, dass sich in der erhaltenen wässrigen Fermentationslösung ein pH-Wert im Bereich von 4,0 bis 7,5, bevorzugt von 5,0 bis 7,0 einstellt. Erforderlichenfalls kann der pH-Wert durch Zugabe von wässrigem oder gasförmigen Ammoniak, wässrigem Kaliumhydroxid oder wässrigem Natriumhydroxid (bei zu niedrigen pH-Werten) bzw. von Salzsäure, Schwefelsäure oder Salpetersäure (bei zu hohen pH-Werten) reguliert werden. Für unterschiedliche Mikroorganismen können unterschiedliche pH-Bereiche innerhalb der genannten Bereiche besonders optimal sein; dies wird im Folgenden näher ausgeführt.

Bevorzugte Mikroorganismen für die Durchführung von Schritt (I) sind **Bakterien** oder **Pilze,** und zwar bevorzugt **Hefen.** Besonders bevorzugt sind dabei Mikroorganismen einer Art ausgewählt aus *Escherichia coli, Pseudomonas putida, Corynebacterium glutamicum, Ashbya gossypii, Pichia pastoris, Hansenula polymorpha, Yarrowia lipolytica, Zygosaccharomyces bailii und Saccharomyces cerevisiae,* wobei bevorzugt neben diesen keine weiteren Mikroorganismen eingesetzt werden. Ganz besonders bevorzugt bestehen die in Schritt (I) eingesetzten Mikroorgansimen nur aus Vertretern genau einer dieser Arten, wobei *Corynebacterium glutamicum* ATTC 13032 außerordentlich ganz besonders bevorzugt ist. Der in der Fermentation einzuhaltende pH-Wert richtet sich wie bereits erwähnt nach dem eingesetzten Mikroorganismus. Mikroorganismen wie *Corynebacterium glutamicum, Pseudomonas putida* oder *Escherichia coli* werden bevorzugt bei "neutralen pH-Werten" (d. h. bei einem pH-Wert im Bereich von 6,0 bis 7,5, bevorzugt 6,0 bis 7,0) kultiviert. Mikroorganismen wie *Saccharomyces cerevisiae* werden hingegen bevorzugt im sauren Milieu kultiviert (d. h. bei einem pH-Wert im Bereich von 4,0 bis 6,0, bevorzugt 5,0 bis 6,0).

In jedem Fall wird der Mikroorganismus aus Schritt (I) bevorzugt so ausgewählt, dass in der Fermentation das ortho-Isomer von Aminobenzoesäure gebildet wird.

In einer bevorzugten Ausgestaltung der Erfindung werden **Bakterien als Mikroorganismen** eingesetzt. Dabei wird insbesondere auf die Patentanmeldungen WO 2015/124686 A1 und WO 2015/124687 A1 verwiesen, in denen Fermentationsprozesse unter Einsatz von Bakterien beschrieben sind (siehe beispielsweise WO 2015/124687 A1, (i) Seite 15, Zeile 8 bis Seite 16, Zeile 30, (ii) Beispiel 1 (Seite 29, Zeile 4 bis 26), (iii) Beispiel 3 (vor allem Seite 34, Zeile 10 bis 18), (iv) Beispiel 4 (vor allem Seite 55, Zeile 9 bis 31). Bevorzugt kommen Bakterien zum Einsatz, die in der Lage sind, eine fermentierbare Kohlenstoff-haltige Verbindung in Gegenwart einer geeigneten Stickstoffquelle in Aminobenzoesäure umzuwandeln, ohne dass die so gebildete Aminobenzoesäure in zellinternen biochemischen Prozessen gleich wieder verbraucht wird, sodass sich Aminobenzoesäure in der Zelle anreichert und schließlich in die Fermentationsbrühe übergeht.

In einer anderen bevorzugten Ausgestaltung der Erfindung werden **Hefen als Mikroorganismen** eingesetzt. Dabei wird insbesondere auf die internationale Anmeldung WO 2017/102853 A1 verwiesen. Insbesondere kommen Hefezellen zum Einsatz, die in der Lage sind, eine fermentierbare Kohlenstoff-haltige Verbindung in Gegenwart einer geeigneten Stickstoffquelle in Aminobenzoesäure umzuwandeln, ohne dass die so gebildete Aminobenzoesäure in zellinternen biochemischen Prozessen gleich wieder verbraucht wird, sodass sich Aminobenzoesäure in der Zelle anreichert und schließlich in die Fermentationsbrühe übergeht. Hefen werden bevorzugt im sauren Milieu kultiviert (d. h. im erfindungsgemäßen Verfahren bei einem pH-Wert im Bereich von 4,0 bis 6,0, bevorzugt 5,0 bis 6,0).

Um ein derartiges Bakterium oder eine derartige Hefe zu erhalten, stehen grundsätzlich zwei Wege zur Verfügung, die in bevorzugter Ausgestaltung auch kombiniert werden können:
(i) Die enzymatischen Reaktionen im Aminobenzoesäure-Stoffwechselweg der Bakterienzelle oder Hefezelle können so erhöht werden, dass Aminobenzoesäure schneller produziert als verbraucht wird.
(ii) Die Folgereaktionen, durch welche Aminobenzoesäure in weitere Metaboliten oder Produkte (z. B. Tryptophan) überführt wird, können reduziert bzw. ausgeschaltet werden, sodass sogar die Geschwindigkeit der Aminobenzoesäure-Bildung in Wildtyp-Stämmen ausreichend ist, um zu einer Anreicherung von Aminobenzoesäure in der Zelle zu führen.

Verfahren zur Gewinnung von Bakterien oder Hefezellen mit den zuvor genannten Eigenschaften sind im Stand der Technik bekannt. Geeignete Bakterien oder Hefezellen können beispielsweise durch Screening nach Mutanten, welche Aminobenzoesäure in das umgebende Medium abgeben, identifiziert werden. Die zielgerichtete Modifikation von Schlüsselenzymen mittels gentechnischer Verfahren ist jedoch bevorzugt. Mit üblichen gentechnischen Methoden können Genexpression und Enzymaktivität nach Belieben verstärkt, verringert oder sogar ganz unterbunden werden. Es resultieren rekombinante Stämme.

Besonders bevorzugt enthalten die Bakterien oder Hefezellen, die in der Lage sind, eine fermentierbare Kohlenstoff-haltige Verbindung in Gegenwart einer Stickstoff-haltigen Verbindung zu Aminobenzoesäure umzusetzen, eine Modifizierung der Anthranilat-Phosphoribosyltransferase-Aktivität, welche besagte Enzymaktivität herabsetzt. Durch diese Modifizierung wird die Umwandlung von ortho-Aminobenzoat zu N-(5-Phospho-D-Ribosyl)-Anthranilat reduziert oder komplett unterbunden. Hierdurch wird eine Anreicherung von Aminobenzoesäure in der Zelle bewirkt. Die Bezeichnung "Anthranilat-Phosphoribosyltransferase-Aktivität" bezieht sich dabei auf eine Enzymaktivität, durch welche die Umsetzung von ortho-Aminobenzoat zu N-(5-Phospho-D-Ribosyl)-Anthranilat katalysiert wird.

In Hefen wird die Anthranilat-Phosphoribosyltransferase-Aktivität durch das native Gen TRP4 (YDR354W) genetisch kodiert. In dem Bakterium *Corynebacterium glutamicum* wird die Anthranilat-Phosphoribosyltransferase-Aktivität durch das *trpD* Gen (cg3361, Cgl3032, NCg12929) kodiert. Im Falle von *Pseudomonas putida* erfolgt die Kodierung über das *trpD* Gen (PP_0421) innerhalb des *trpDC* Operons.

Die beschriebene Herabsetzung der Anthranilat-Phosphoribosyltransferase-Aktivität kann prinzipiell auf drei Wegen erreicht werden:
(i) Die Regulierung der Expression des Gens für die Anthranilat-Phosphoribosyltransferase-Aktivität kann so modifiziert werden, dass die Transkription des Gens oder anschließende Translation verringert oder unterbunden wird.
(ii) Die Nukleinsäuresequenz des Gens für Anthranilat-Phosphoribosyltransferase-Aktivität kann so modifiziert werden, dass das Enzym, welches durch das modifizierte Gen kodiert wird, eine geringere spezifische Aktivität aufweist.
(iii) Das native Gen für Anthranilat-Phosphoribosyltransferase-Aktivität kann durch ein anderes Gen, das von einem verschiedenen Organismus stammt, ersetzt und ein Enzym mit einer spezifischen Anthranilat-Phosphoribosyltransferase-Aktivität, die geringer ist als die der zuvor erwähnten nativen Gene (z.B. TRP4, *trpD* oder *trpDC),* kodiert werden.

Unabhängig davon, welcher Mikroorganismus eingesetzt wird, umfasst die Fermentationsbrühe zu Beginn der Fermentation in Schritt (I) rekombinante Zellen des eingesetzten Mikroorganismus und mindestens eine fermentierbare Kohlenstoff-haltige Verbindung (sowie mindestens eine Stickstoff-haltige Verbindung als Stickstoffquelle). Bevorzugt enthält die Fermentationsbrühe darüber hinaus noch weitere Bestandteile ausgewählt aus der Gruppe bestehend aus Puffersystemen, anorganischen Nährstoffen, Aminosäuren, Vitaminen und weiteren organischen Verbindungen welche für das Wachstum bzw. den Erhaltungsstoffwechsel der rekombinanten Zellen benötigt werden. Die Fermentationsbrühe ist wasserbasiert. Nach Einsetzen des Fermentationsprozesses umfasst die Fermentationsbrühe auch Aminobenzoesäure, das angestrebte Fermentationsprodukt.

Wie bereits erwähnt, wird unter einer fermentierbaren Kohlenstoff-haltigen Verbindung im Sinne der vorliegenden Erfindung jede organische Verbindung oder Mischung organischer Verbindungen verstanden, die von den rekombinanten Zellen des eingesetzten Mikroorganismus verwendet werden kann, um Aminobenzoesäure zu produzieren. Die Produktion von Aminobenzoesäure kann dabei in Gegenwart oder Abwesenheit von Sauerstoff stattfinden.

Bevorzugt sind dabei solche fermentierbaren Kohlenstoff-haltigen Verbindungen, die zusätzlich als Energie- und Kohlenstoffquelle für das Wachstum der rekombinanten Zellen des eingesetzten Mikroorganismus dienen können. Besonders geeignet sind Stärkehydrolysat, Zuckerrohrsaft, Zuckerrübensaft und Hydrolysate aus lignocellulosehaltigen Rohstoffen. Ebenfalls geeignet sind Glycerin und C1-Verbindungen, bevorzugt Kohlenstoffmonoxid.

Unabhängig vom eingesetzten Mikroorganismus und der gewählten Kohlenstoff- und Stickstoffquelle wird das in Schritt (I) einzusetzende anorganische Calcium-Salz bevorzugt ausgewählt aus Calciumcarbonat, Calciumhydrogencarbonat, Calciumhydroxid, Calciumoxid oder Mischungen aus zwei oder mehr der vorgenannten Verbindungen. Insbesondere bevorzugt ist der Einsatz einer Mischung aus Calciumcarbonat und Calciumhydroxid. Eine Suspension von Calciumcarbonat in Wasser enthält - da Calciumcarbonat zu einem Teil in Lösung geht und die gelösten Carbonationen mit Wasser Hydrogencarbonat- und Hydroxidionen ausbilden - immer auch Anteile an Calciumhydroxid und ist daher von der Formulierung "Mischung aus Calciumcarbonat und Calciumhydroxid" umfasst. Der Einsatz solcher Calcium-Salze hat den Vorteil, dass die Zugabe von weiteren Basen wie beispielsweise Natriumhydroxid (siehe etwa die in WO 2015/124686 A1 und WO 2015/124687 A1 beschriebenen Verfahren) als Puffer nicht mehr oder allenfalls in verringerter Menge erforderlich ist. Calciumcarbonat kann dabei in fester Form im Fermentationsreaktor vorgelegt werden. Eine Zugabe als wässrige Suspension ist auch möglich. Calciumoxid kann grundsätzlich ebenfalls in fester Form in den Fermentationsreaktor eingeführt werden. Steht Calciumoxid als Calciumquelle zur Verfügung, so ist es jedoch bevorzugt, dieses zunächst mit Wasser abzulöschen und so in Calciumhydroxid zu überführen. Calciumhydroxid und Calciumhydrogencarbonat werden vorzugsweise in Form von wässrigen Lösungen zudosiert.

Die erfindungsgemäße Fermentation (Schritt (I)) wird, wie bereits erwähnt, bevorzugt bei pH-Werten im Bereich von 4,0 bis 7,5, bevorzugt 5,0 bis 7,0 durchgeführt. Unter diesen pH-Bedingungen können auch aus Calcium-Salzen, die unter anderen Bedingungen unlöslich sind, Calcium-Ionen zumindest teilweise in Lösung gehen, die dann in der Lage sind, gebildetes Aminobenzoat zu komplexieren und auszufällen. Auf diese Weise gehen dann auch Calcium-Salze wie Calciumcarbonat nach und nach in Lösung (wobei im Fall von Calcium-Carbonat Kohlenstoffdioxid aus den Carbonat-Ionen gebildet wird, welches - zumindest teilweise - ausgast).

Das in Schritt (I) einzusetzende anorganische Calcium-Salz wird vorzugsweise in, bezogen auf die zu erwartende Menge an produzierter Aminobenzoesäure, mindestens stöchiometrischer Menge verwendet. Auch der Einsatz eines Überschusses, beispielsweise ein molares Verhältnis von Calcium-Ionen zur erwarteten Menge an produzierter Aminobenzoesäure von 1 : 1 oder mehr ist möglich.

In einer Ausführungsform der Erfindung wird Schritt (I) kontinuierlich durchgeführt, d. h. dass die Edukte dem Fermentationsreaktor kontinuierlich zugeführt und das Produkt dem Fermentationsreaktor kontinuierlich entnommen wird. Das dem Fermentationsreaktor kontinuierlich entnommene Produkt ist dabei im einfachsten Fall das in der wässrigen Fermentationslösung suspendierte Gemisch umfassend nicht gelöste Mikroorganismen und ausgefallenes Calcium-Aminobenzoat, also die Fermentationsbrühe. Es ist jedoch auch denkbar, durch Anwendung geeigneter Trennverfahren die Mikroorganismen selektiv im Fermentationsreaktor zurückzuhalten und nur eine wässrige Suspension von Calcium-Aminobenzoat auszuschleusen. Eine solche Trennung erfolgt vorzugsweise durch Ausnutzung der unterschiedlichen Dichten von Calcium-Aminobenzoat und Mikroorganismen, beispielsweise in einem Flotationsverfahren, wobei dann die Mikroorganismen oben "aufschwimmen", oder unter Anwendung von Zentrifugation.

In einer anderen Ausführungsform der Erfindung wird Schritt (I) in einer diskontinuierlichen Verfahrensführung (sog. "Batch-Fahrweise") in *Fermentationszyklen* durchgeführt. Dabei umfasst ein Fermentationszyklus bevorzugt die initiale Vorlage oder Zugabe von Mikroorganismen in ein Nährmedium, die Vorlage und/oder Zugabe von Nährstoffen, den Aufbau von Mikroorganismen, die Bildung des gewünschten Produktes, also der Aminobenzoesäure, sowie die vollständige oder teilweise Entleerung des Reaktors nach Abschluss der Fermentation. In einer Variante der diskontinuierlichen Fahrweise (sog. "*Fed*-Batch-Fahrweise") werden die Edukte dem Fermentationsreaktor so lange kontinuierlich zugeführt, wie es das Reaktorvolumen erlaubt, ohne dass Produkte - gegebenenfalls ausgenommen gasförmige Bestandteile, die über eine Anbindung des Reaktors an ein Abgassystem ausgetragen werden - dem Reaktor entnommen werden. Die Reaktion wird nach Zugabe der maximal möglichen Menge an Edukten unterbrochen und das Produktgemisch dem Fermentationsreaktor entnommen. Details zu diesen an dieser Stelle nur kurz umrissenen Fahrweisen werden weiter unten erläutert.

Unabhängig von der genauen Fahrweise umfasst der Fermentationsreaktor bevorzugt Einrichtungen zur Messung wichtiger Prozessparameter wie Temperatur, pH-Wert, Konzentration an Substrat und Produkt, Gehalt an gelöstem Sauerstoff, Zelldichte der Fermentationsbrühe. Insbesondere bevorzugt umfasst der Fermentationsreaktor Einrichtungen zur Anpassung von mindestens einem (bevorzugt von allen) der vorgenannten Prozessparameter.

Geeignete Fermentationsreaktoren sind Rührkessel, Membranreaktoren, Kolbenstromreaktoren ("plug flow reactors") oder Schlaufenreaktoren (siehe beispielsweise Bioprozesstechnik, Horst Chmiel, ISBN-10: 3827424763, Spektrum Akademischer Verlag). Besonders bevorzugt sowohl für aerobe als auch für anaerobe Fermentationen sind Rührkesselreaktoren und Schlaufenreaktoren (bevorzugt Airliftreaktoren, in denen die Zirkulation der Flüssigkeit im Reaktor durch Begasung erreicht wird).

In **Schritt (II)** des erfindungsgemäßen Verfahrens wird das in Schritt (I) ausgefallene Calcium-Aminobenzoat aus der wässrigen Fermentationslösung zunächst in einem ersten Schritt (II)(1) **isoliert.** Dies geschieht entweder selektiv, d. h. unter Abtrennung auch der nicht gelösten Mikroorganismen, oder nicht selektiv, d. h. das Gemisch der festen Bestandteile der Fermentationsbrühe enthaltend die nicht gelösten Mikroorganismen und ausgefallenes Calcium-Aminobenzoat wird als unveränderte Gesamtheit von der wässrigen Fermentationslösung abgetrennt. Letzteres kann durch eine einfache Filtration, Sedimentation oder Zentrifugation bewirkt werden. Die selektive Trennung ist beispielsweise unter Ausnutzung der unterschiedlichen Dichten von Calcium-Aminobenzoat und Mikroorganismen möglich, etwa in einem Flotationsverfahren, wobei dann die Mikroorganismen oben "aufschwimmen", oder in einem Zentrifugationsverfahren. Wie bereits im Zusammenhang mit Schritt (I) erläutert, kann eine solche Abtrennung von Mikroorganismen von Calcium-Aminobenzoat auch derart erfolgen, dass die Mikroorganismen im Fermentationsreaktor zurückgehalten werden.

Die in Schritt (II)(1) nach der Isolierung des Calcium-Aminobenzoats verbleibende wässrige Fermentationslösung weist einen pH-Wert von bevorzugt 4,0 oder mehr auf, abhängig von den genauen Bedingungen der Fermentation. Aus dieser wässrigen Fermentationslösung kann, da sie noch Anteile gelösten Aminobenzoats enthalten kann, in vorteilhafter Weise durch Säurezugabe bis zum Erreichen eines pH-Werts im Bereich von 3,0 bis < 4,0 Aminobenzoesäure auskristallisiert und die auskristallisierte Aminobenzoesäure isoliert werden, wobei eine an Aminobenzoesäure abgereicherte Mutterlauge verbleibt. Als Säure eignen sich hierfür bevorzugt Mineralsäuren wie Schwefel-, Phosphor- oder Salzsäure; Salzsäure und Schwefelsäure sind besonders bevorzugt. Die erhaltene Mutterlauge enthält immer noch Restanteile an Aminobenzoesäure. Es ist daher bevorzugt, diese Mutterlauge durch eine Folge aus einem Adsorptionsschritt und einem Desorptionsschritt aufzukonzentrieren. Dabei kann der Desorptionsschritt durch Elution mit einem Desorptionsmittel eines pH-Werts im Bereich von 6,0 bis 11,0 durchgeführt und das so erhaltene Desorbat vorteilhaft als Bestandteil der in Schritt (II)(2) zugegebenen wässrigen Phase eingesetzt werden. In einer anderen Ausführungsform wird die Desorption in einem stark sauren Bereich durchgeführt (d. h. das Desorptionsmittel weist einen pH-Wert von weniger als 3,0 auf), und das Desorbat wird einer Nachkristallisation durch Erhöhung des pH-Werts auf einen Wert ≥ 3,0 bevorzugt auf einen Wert im Bereich von 3,0 bis < 4,0 unterzogen. Als im Adsorptionsschritt einsetzbares Adsorptionsmittel eignet sich beispielsweise Aktivkohle.

Es kann sinnvoll sein, die in Schritt (II)(1) nach der Isolierung des Calcium-Aminobenzoats verbleibende wässrige Fermentationslösung vor der Kristallisation und/oder die in der Kristallisation erhaltene Mutterlauge vor der Adsorption zu *entfärben.* Diese Entfärbung wird in einer Ausführungsform bevorzugt so durchgeführt, dass die wässrige Fermentationslösung bzw. die Mutterlauge über eine Säule mit fester Packung geleitet wird, um Farbstoffe mittels Adsorption zu entfernen. Als mögliche feste Phase kann z. B. Kieselgur, Aktivkohle oder eine Ionenaustauscherpackung verwendet werden. In einer anderen Ausführungsform wird die Entfärbung durch Rühren mit pulverförmiger Aktivkohle oder pulverförmigen Kieselgur gefolgt von Filtration durchgeführt.

Grundsätzlich ist es anstelle einer Kristallisation auch vorstellbar, die in Schritt (II)(1) nach der Isolierung des Calcium-Aminobenzoats verbleibende wässrige Fermentationslösung anteilig in die Fermentation aus Schritt (I) zurückzuführen. Wegen der damit einhergehenden Verdünnung der Reaktionsmischung ist jedoch im Allgemeinen die Ausführungsform unter Kristallisation zu bevorzugen.

In Schritt (II)(2) wird das im in Schritt (II)(1) isolierten Calcium-Aminobenzoat gebundene Aminobenzoat durch Überführung in eine wasserlösliche Form freigesetzt **(Ionenaustauschschritt).** Dies geschieht erfindungsgemäß, indem das in Schritt (II)(1) erhaltene Verfahrensprodukt (also entweder Calcium-Aminobenzoat oder ein Gemisch enthaltend Calcium-Aminobenzoat und nicht gelöste Mikroorganismen) mit einer wässrigen Phase umfassend
Lithium-, Natrium-, Kalium- und/oder Ammonium-Kationen, bevorzugt Ammonium-Kationen, und
Carbonat- und/oder Hydrogencarbonat-Anionen,
versetzt wird. Besonders bevorzugt ist dabei eine Lösung von Ammoniumcarbonat. Dabei geht Aminobenzoat als Salz des eingeführten Kations in Lösung, während das eingeführte Anion als Calcium-Salz ausfällt. Der Ionenaustauschschritt erfolgt üblicherweise bei Umgebungstemperatur innerhalb weniger Minuten. Eine innige Vermischung der zugegebenen wässrigen Phase mit dem Calcium-Aminobenzoat etwa durch intensives Rühren ist vorteilhaft. Es ist nicht erforderlich, dass sämtliches im Calcium-Aminobenzoat gebundenes Aminobenzoat in Schritt (II)(2) gelöst wird; evtl. verbleibende nicht gelöste Restbestandteile können im folgenden Schritt (II)(3) wie dort beschrieben in Schritt (I) rezykliert werden. Dies verlässt den Rahmen der Erfindung nicht.

Schritt (II)(2) wird bevorzugt bei einem pH-Wert von > 7,0, bevorzugt > 8,0, durchgeführt. Sofern sich ein solcher pH-Wert nicht unter den gewählten Prozessbedingungen von alleine einstellt, ist es zweckmäßig, Base, bevorzugt wässrigen oder gasförmigen Ammoniak, zuzugeben.

In Schritt (II)(3) wird das in Schritt (II)(2) erhaltene Verfahrensprodukt (also der ausgefallene Feststoff - entweder das wasserunlösliche Calcium-Salz [(2)(i)] oder das Gemisch enthaltend das wasserunlösliche Calcium-Salz und nicht gelöste Mikroorganismen [(2)(ii)] - suspendiert in einer wässrigen Lösung, die das in Lösung gegangene Aminobenzoat enthält) in seine festen und flüssigen Bestandteile getrennt, wobei gegebenenfalls auch eine Trennung von Calcium-Salz [(2)(i)] und nicht gelösten Mikroorganismen [(2)(ii)] vorgenommen werden kann. In jedem Fall können die gleichen Trenntechniken wie zuvor für Schritt (II)(1) beschrieben, eingesetzt werden. In allen Ausführungsformen der Erfindung ist es bevorzugt, den in Schritt (II)(3) abgetrennten Feststoff in Schritt (I) zurückzuführen, da er dort in vorteilhafter Weise zur Bereitstellung des Calcium-Salzes dienen kann. Wenn im vorangegangenen Schritt nicht sämtliches im Calcium-Aminobenzoat gebundenes Aminobenzoat in Lösung gegangen ist, so verbleibt nicht gelöstes Calcium-Aminobenzoat in diesem Feststoff und wird gemeinsam mit diesem in Schritt (I) zurückgeführt, sodass keine Verluste auftreten.

In **Schritt (III)** erfolgt die Abscheidung von Aminobenzoesäure durch Einleiten von Kohlenstoffdioxid unter erhöhtem Druck in die in Schritt (II)(3) erhaltene wässrige Lösung von Aminobenzoat **(Kristallisationsschritt).** Durch das Einpressen von Kohlenstoffdioxid kommt es infolge der Ausbildung von Protolysegleichgewichten zu einer Erniedrigung des pH-Werts in einen Bereich, in dem Aminobenzoat in Aminobenzoesäure überführt wird, welche aufgrund ihrer geringen Löslichkeit in wässrigen Medien ausfällt. Bevorzugt erfolgt daher das Einleiten von Kohlenstoffdioxid so lange, bis sich ein pH-Wert von 5,0 oder weniger eingestellt hat. Eine quantitative Abscheidung von Aminobenzoesäure an dieser Stelle ist aufgrund der erfindungsgemäßen Rückführung von Schritt (V) nicht unbedingt erforderlich. In bevorzugter Ausgestaltung der Erfindung wird wie folgt verfahren:
Die in Schritt (II)(3) erhaltene wässrige Lösung von Aminobenzoat wird mit Kohlenstoffdioxid versetzt, und zwar insbesondere derart, dass nur ein Teil, bevorzugt 5,0 % bis 90 % des Aminobenzoats als Säure auskristallisieren. Die ausgefallene Säure wird durch Filtration, bevorzugt bei dem gleichen Druck, unter dem das Kohlenstoffdioxid eingepresst wurde (Druckfiltration), isoliert. Das verbleibende Filtrat wird entspannt (vorzugsweise auf Umgebungsdruck), wobei Kohlenstoffdioxid teilweise ausgast. Das Filtrat enthält auch nach der Entspannung noch Carbonationen und Restanteile an Aminobenzoat (mit dem in Schritt (II)(2) eingeführten wasserlösliche Aminobenzoat-Salze bildenden Kation als Gegenion). Dieses Filtrat wird wieder in den Ionenaustauschschritt (II)(2) geführt. Da dem Schritt (II)(2) über den Schritt (II)(1) stets neues Calcium-Aminobenzoat zugeführt wird, wird auf diese Weise der durch Auskristallisation entfernte Anteil an Aminobenzoesäure ersetzt.

In **Schritt (IV)** wird die in Schritt (III) auskristallisierte Aminobenzoesäure abgetrennt **(Isolierungsschritt).** Schritt (IV) umfasst auch eine Druckerniedrigung unter Freisetzung von Kohlenstoffdioxid, sodass als Verfahrensprodukt dieses Schrittes eine an Kohlenstoffdioxid abgereicherte und von *abgeschiedener* Aminobenzoesäure befreite wässrige Lösung erhalten wird. Wie bereits erwähnt, wird im vorangegangenen Schritt nicht notwendigerweise sämtliches Aminobenzoat als Säure ausgefällt. In Lösung verbliebene Aminobenzoationen werden in Schritt (IV) nicht mit abgetrennt.

Unabhängig von der genauen Ausgestaltung dieses Schrittes fällt Kohlenstoffdioxid an, welches vorteilhafterweise aufgefangen und für eine erneute Zugabe in Schritt (III) eingesetzt werden kann. Dies führt zu einer wirtschaftlich und ökologisch sehr vorteilhaften Kreislaufführung.

Bevorzugt umfasst dieser Schritt (IV) folgende Teilschritte:
(1) Trennen der Aminobenzoesäure und der wässrigen Lösung aus Schritt (III) bei einem Druck gleich dem oder größer als der Druck in Schritt (III),
(2) Entspannen der in Schritt (1) abgetrennten wässrigen Lösung unter Freisetzung von Kohlenstoffdioxid, wobei eine an Kohlenstoffdioxid abgereicherte wässrige Lösung erhalten wird. Das bei der Entspannung freiwerdende Kohlenstoffdioxid kann aufgefangen und für eine erneute Zugabe in Schritt (III) eingesetzt werden.

In Schritt (1) wird die im vorigen Schritt abgeschiedene Aminobenzoesäure von der wässrigen Lösung abgetrennt. Methoden hierzu sind an sich aus dem Stand der Technik bekannt. Erfindungsgemäß erfolgt dieser Schritt bevorzugt durch Filtration oder Zentrifugation. Bevorzugt wird dieser Schritt durchgeführt wie in WO 2015/124687 A1 beschrieben. Dabei wird insbesondere auf WO 2015/124687 A1, Seite 17, Zeile 13 bis Seite 17, Zeile 16, verwiesen. Die Filtration erfolgt dabei bei erhöhtem Druck, um ein frühzeitige Entgasen von Kohlenstoffdioxid zu vermeiden. Eine Zentrifugation kann mit handelsüblichen Zentrifugen (ebenfalls unter Druck) durchgeführt werden. Es ist auch möglich, die im ersten Schritt erhaltene Suspension ruhen zu lassen, bis sich die ausgefallenen Kristalle an Aminobenzoesäure absetzen, um dann die überstehende Mutterlauge unter Druck zu dekantieren oder abzusaugen.

Die so gewonnene Aminobenzoesäure kann optional weiter gereinigt werden. Auch dieser Teilschritt von Schritt (IV) ist an sich aus dem Stand der Technik bekannt (siehe vor allem WO 2015/124687 A1 und insbesondere auf WO 2015/124687 A1, Seite 18, Zeile 4 bis Seite 18, Zeile 6) und erfolgt bevorzugt durch eine oder mehrere Wäschen mit wässrigen Waschmedien, bevorzugt Wasser. Um Ausbeuteverluste zu vermeiden, wird der pH-Wert des wässrigen Waschmediums vorzugsweise auf einen Wert im Bereich von 3,0 bis < 4,0, bevorzugt auf einen Wert von 3,5, eingestellt.

In **Schritt** (V) wird die in Schritt (IV) nach der Abtrennung der Aminobenzoesäure verbleibende, von abgeschiedener Aminobenzoesäure befreite wässrige Lösung in den Schritt (II)(2) rezykliert und dort als Bestandteil der in diesem Schritt einzusetzenden wässrigen Phase (**AQ)** verwendet (**Rezyklierungsschritt).** Die Formulierung *"wird* ... *als Bestandteil der in diesem Schritt einzusetzenden wässrigen Phase verwendet"* umfasst auch den Fall, dass die in Schritt (IV) nach der Abtrennung der Aminobenzoesäure verbleibende, von abgeschiedener Aminobenzoesäure befreite wässrige Lösung der alleinige Bestandteil der in Schritt (II)(2) einzusetzenden wässrigen Phase ist. Es ist also erfindungsgemäß möglich, dass - abgesehen von Abweichungen vom regulären Betrieb des Verfahrens, z. B. bei Anfahrvorgängen -wässrige Phase **(AQ)** enthaltend wasserlösliche Aminobenzoat-Salze bildende Kationen und wasserunlösliche Calcium-Salze *allein durch diese Rezyklierung gemäß Schritt (V) bereitgestellt wird.* Eventuell erforderliche pH-Wert-Anpassungen, wie sie weiter oben im Zusammenhang mit der Diskussion des Schritts (II)(2) erläutert wurden, bleiben hiervon natürlich unberührt; sind also auch dann möglich, wenn die wässrige Phase AQ nur durch Rezyklierung bereitgestellt wird.

Das erfindungsgemäße Verfahren ermöglicht also eine äußerst vorteilhafte Kreislaufführung.

Im Folgenden werden besonders vorteilhafte verfahrenstechnische Ausgestaltungen des erfindungsgemäßen Verfahrens geschildert:

### DISKONTINUIERLICHE VERFAHRENSFÜHRUNG

In einer ersten Variante der bereits weiter oben erwähnten *diskontinuierlichen* Verfahrensführung wird nach Abschluss eines Fermentationszyklus
Schritt (II)(1) durchgeführt, indem die in Schritt (I) erhaltene wässrige Fermentationslösung unter Zurückhaltung des in dieser suspendierten Gemisches umfassend nicht gelöste Mikroorganismen und ausgefallenes Calcium-Aminobenzoat aus dem Fermentationsreaktor ausgeschleust wird;
Schritt (II)(2) durchgeführt, indem die wässrige Phase in den Fermentationsreaktor gegeben wird, sodass im Fermentationsreaktor eine Suspension enthaltend ein Gemisch umfassend nicht gelöste Mikroorganismen und das wasserunlösliche Calcium-Salz in einer wässrigen Lösung von Aminobenzoat erhalten wird;
und
Schritt (II)(3) durchgeführt, indem die in Schritt (II)(2) erhaltene wässrige Lösung von Aminobenzoat aus dem Fermentationsreaktor ausgeschleust wird, wobei das Gemisch umfassend nicht gelöste Mikroorganismen und das wasserunlösliche Calcium-Salz zurückgehalten wird und für den nächsten Fermentationszyklus zur Verfügung steht.

In einer zweiten Variante der diskontinuierlichen Verfahrensführung wird nach Abschluss eines Fermentationszyklus
Schritt (II)(1) durchgeführt, indem die in Schritt (I) erhaltene wässrige Fermentationslösung *gemeinsam* mit dem in dieser suspendierten Gemisch umfassend nicht gelöste Mikroorganismen und ausgefallenes Calcium-Aminobenzoat aus dem Fermentationsreaktor ausgeschleust wird und das Gemisch umfassend nicht gelöste Mikroorganismen und ausgefallenes Calcium-Aminobenzoat außerhalb des Fermentationsreaktors von der wässrigen Fermentationslösung getrennt und anschließend das so abgetrennte Feststoff-Gemisch in den Fermentationsreaktor zurückgeführt wird;
Schritt (II)(2) durchgeführt, indem die wässrige Phase in den Fermentationsreaktor gegeben wird, sodass im Fermentationsreaktor eine Suspension enthaltend ein Gemisch umfassend nicht gelöste Mikroorganismen und das wasserunlösliche Calcium-Salz in einer wässrigen Lösung von Aminobenzoat erhalten wird;
und
Schritt (II)(3) durchgeführt, indem die in Schritt (II)(2) erhaltene wässrige Lösung von Aminobenzoat aus dem Fermentationsreaktor ausgeschleust wird, wobei das Gemisch umfassend nicht gelöste Mikroorganismen und das wasserunlösliche Calcium-Salz zurückgehalten wird und für den nächsten Fermentationszyklus zur Verfügung steht.

Schließlich wird in einer dritten Variante der diskontinuierlichen Verfahrensführung nach Abschluss eines Fermentationszyklus
Schritt (II)(1) durchgeführt, indem die in Schritt (I) erhaltene wässrige Fermentationslösung gemeinsam mit dem in dieser suspendierten Gemisch umfassend nicht gelöste Mikroorganismen und ausgefallenes Calcium-Aminobenzoat aus dem Fermentationsreaktor ausgeschleust wird und das Gemisch umfassend nicht gelöste Mikroorganismen und ausgefallenes Calcium-Aminobenzoat außerhalb des Fermentationsreaktors von der wässrigen Fermentationslösung getrennt und in einen vom Fermentationsreaktor verschiedenen Behälter gegeben wird;
Schritt (II)(2) durchgeführt, indem die wässrige Phase in diesen Behälter gegeben wird, sodass in diesem Behälter eine Suspension enthaltend ein Gemisch umfassend nicht gelöste Mikroorganismen und das wasserunlösliche Calcium-Salz in einer wässrigen Lösung von Aminobenzoat erhalten wird;
wobei ferner
nach der Abtrennung der in Schritt (II)(2) erhaltenen wässrigen Lösung von Aminobenzoat von dem Gemisch umfassend nicht gelöste Mikroorganismen und das wasserunlösliche Calcium-Salz in Schritt (II)(3) dieses abgetrennte Gemisch in einem Schritt (II)(4) wieder in den Fermentationsreaktor gegeben wird, sodass es für den nächsten Fermentationszyklus zur Verfügung steht.

Unabhängig von der genauen Ausgestaltung des diskontinuierlichen Verfahrens ist es bevorzugt, die Schritte (I) und (II) solange zu wiederholen, bis in Schritt (IV) die gewünschte Menge an Aminobenzoesäure erhalten wird oder die in Schritt (I) eingesetzten Mikroorganismen infolge ihrer Erschöpfung erneuert werden müssen. Diese Fahrweise kann sowohl als "Batch-Fahrweise" (infolge der Wiederholungen der Einzelschritte genauer: *"Repeated* Batch-Fahrweise") als auch als "Fed-Batch-Fahrweise" (infolge der Wiederholungen der Einzelschritte genauer: *"Repeated* Fed-Batch-Fahrweise") betrieben werden.

### KONTINUIERLICHE VERFAHRENSFÜHRUNG

In einer ersten Variante der bereits weiter oben erwähnten *kontinuierlichen* Verfahrensführung wird dem Fermentationsreaktor kontinuierlich Fermentationsbrühe, also das in der wässrigen Fermentationslösung suspendierte Gemisch umfassend nicht gelöste Mikroorganismen und ausgefallenes Calcium-Aminobenzoat, entnommen. Dabei wird
Schritt (II)(1) durchgeführt, indem nach erfolgter Ausschleusung von Fermentationsbrühe die unlöslichen Mikroorganismen und ausgefallenes Calcium-Aminobenzoat voneinander und von der wässrigen Fermentationslösung getrennt werden;
Schritt (II)(2) durchgeführt, indem die wässrige Phase zu dem so abgetrennten Calcium-Aminobenzoat gegeben wird;
wobei ferner die in Schritt (II)(1) abgetrennten unlöslichen Mikroorganismen teilweise bis vollständig in den Fermentationsreaktor zurückgeführt werden.

In einer zweiten Variante der bereits weiter oben erwähnten *kontinuierlichen* Verfahrensführung wird dem Fermentationsreaktor kontinuierlich in der wässrigen Fermentationslösung suspendiertes ausgefallenes Calcium-Aminobenzoat, ohne die unlöslichen Mikroorganismen, entnommen, d. h. in Schritt (II)(1) werden zunächst nur die Mikroorganismen abgetrennt und bleiben im Fermentationsreaktor. Nach erfolgter Ausschleusung der Suspension aus Calcium-Aminobenzoat in wässriger Fermentationslösung wird dann das ausgefallene Calcium-Aminobezoat von der wässrigen Fermentationslösung getrennt. Schritt (II)(2) wird durchgeführt, indem die wässrige Phase zu dem so abgetrennten Calcium-Aminobenzoat gegeben wird. Die zweite Variante ist also eine Variante der ersten Variante, die sich darin unterscheidet, dass die Trennung von Mikroorganismen und Calcium-Aminobenzoat bereits im Fermentationsreaktor durchgeführt wird.

In einer dritten Variante der bereits weiter oben erwähnten kontinuierlichen Verfahrensführung wird dem Fermentationsreaktor wie in der ersten Variante kontinuierlich Fermentationsbrühe, also das in der wässrigen Fermentationslösung suspendierte Gemisch umfassend nicht gelöste Mikroorganismen und ausgefallenes Calcium-Aminobenzoat, entnommen. Dabei wird
Schritt (II)(1) durchgeführt, indem nach erfolgter Ausschleusung der Fermentationsbrühe das Gemisch umfassend nicht gelöste Mikroorganismen und ausgefallenes Calcium-Aminobenzoat von der wässrigen Fermentationslösung getrennt wird;
Schritt (II)(2) durchgeführt, indem die wässrige Phase zu diesem so abgetrennten Gemisch gegeben wird;
wobei ferner
nach der Abtrennung der in Schritt (II)(2) erhaltenen wässrigen Lösung von Aminobenzoat von dem Gemisch umfassend nicht gelöste Mikroorganismen und das wasserunlösliche Calcium-Salz in Schritt (II)(3) dieses abgetrennte Gemisch in einem Schritt (II)(4) wieder in den Fermentationsreaktor gegeben wird und für die weitere kontinuierliche Fermentation zur Verfügung steht.

In einer vierten Variante der bereits weiter oben erwähnten kontinuierlichen Verfahrensführung wird zunächst so verfahren wie in der dritten Variante, jedoch wird im Unterschied dazu das in Schritt (II)(3) abgetrennte Gemisch zunächst in einem Schritt (II)(4a) in seine wesentlichen Bestandeile, also in nicht gelöste Mikroorganismen und wasserunlösliches Calcium-Salz, aufgetrennt, und dann wird in einem Schritt (II)(4b) nur einer der so separierten Bestandteile, bevorzugt das wasserunlösliche Calcium-Salz, wieder in den Fermentationsreaktor gegeben. Dies ist insbesondere dann sinnvoll, wenn die Mikroorganismen aus Schritt (II)(3) bereits erschöpft sind und durch frische ersetzt werden müssen.

Im oben beschriebenen Schritt (IV) wird die Aminobenzoesäure in der elektroneutralen Form gewonnen. In dieser Form kann sie unmittelbar der optionalen (und bevorzugten) Umsetzung zu einem Aminobenzoesäure-Folgeprodukt in **Schritt (VI)** zugeführt werden. Diese weiteren Umsetzungen der erhaltenen Aminobenzoesäure in Schritt (VI) sind:
(1) Decarboxylierung der Aminobenzoesäure zu Anilin;
(2) Decarboxylierung der Aminobenzoesäure zu Anilin, gefolgt von säurekatalysierter Reaktion des Anilins mit Formaldehyd unter Bildung von Di- und Polyaminen der Diphenylmethanreihe;
(3) Decarboxylierung der Aminobenzoesäure zu Anilin, gefolgt von säurekatalysierter Reaktion des Anilins mit Formaldehyd unter Bildung von Di- und Polyaminen der Diphenylmethanreihe, gefolgt von Umsetzung mit Phosgen unter Bildung von Di- und Polyisocyanaten der Diphenylmethanreihe;
(4) Decarboxylierung der Aminobenzoesäure zu Anilin, gefolgt von Umsetzung des Anilins zu einer Azoverbindung;
(5) Umsetzung der Aminobenzoesäure zu einem Amid;
(6) Umsetzung der Aminobenzoesäure zu einem leitenden Polymeren wie bevorzugt Polyanthranilsäure.

Die Decarboxylierung der Aminobenzoesäure zu **Anilin (VI)(1)** ist an sich bekannt und kann nach einem beliebigen Verfahren des Standes der Technik durchgeführt werden. Bevorzugt ist die folgende Vorgehensweise:
Schritt (VI)(1) kann in allen in der Verfahrenstechnik üblichen, dem Fachmann geläufigen Reaktortypen wie bspw.
- Rührkesselreaktoren,
- kontinuierlich betriebene Rührkesselreaktoren,
- Rohrreaktoren, bevorzugt mit Katalysatorfestbett oder
- Schlammphasenreaktoren (auch Suspensionsreaktoren genannt; in der englischen Literatur als *slurry phase reactors* bezeichnet) mit Katalysator-Rezirkulation bzw. Katalysator-Rückgewinnung
durchgeführt werden.

Es können auch mehrere Reaktoren zu einer Reaktorkaskade hintereinandergeschaltet werden, d. h. der flüssige Produktaustrag eines Reaktors fließt zur weiteren Umsatzvervollständigung in den nächstfolgenden Reaktor.

Die Decarboxylierung von Schritt (VI)(1) gelingt in Gegenwart eines Katalysators leicht. Als Katalysatoren für die Durchführung von Schritt (VI)(1) eignen sich dem Fachmann geläufige Katalysatoren wie bspw. wässrige Säuren wie Schwefelsäure, Salpetersäure und Salzsäure; feste Säuren wie Zeolithe und Si-Ti-Molekularsiebe, feste Basen wie Hydroxy-Apatite und Hydrotalcite; polymere Säuren wie Ionenaustauscherharze (bevorzugt Amberlyst). Wird der Katalysator in Form von Partikeln oder in Pulverform eingesetzt, so besteht eine bevorzugte Ausführungsform der Erfindung darin, den Katalysator in der flüssigen Reaktionsmischung aufzuschlämmen, bevorzugt durch Rühren. Hierzu eignet sich besonders ein Schlammphasenreaktor (auch Suspensionsreaktor genannt), wobei der Katalysator in einer Konzentration im Bereich von 0,100 Massen-% bis 50,0 Massen-%, bevorzugt im Bereich von 10,0 Massen-% bis 30,0 Massen-%, bezogen auf die Gesamtmasse der flüssigen Reaktionsmischung, eingesetzt wird. In einer anderen bevorzugten Ausführungsform wird der Katalysator in einem Katalysatorbett in einem Rohrreaktor angeordnet, wobei der in dieser Ausführungsform bevorzugt in Partikeln (bspw. Kugeln) vorliegende Katalysator vorzugsweise im Katalysatorbett fixiert ist, bspw. zwischen Siebplatten angeordnet ist. Unabhängig von der Art des eingesetzten Reaktors wird als in Schritt (VI)(1) eingesetzter Katalysator vorzugsweise ein Zeolith-Katalysator verwendet, besonders bevorzugt ein Zeolith vom Typ Y in der protonierten Form (H-Form). Die Anordnung des, bevorzugt in Partikelform vorliegenden, Katalysators in einem fixierten Bett ist natürlich nicht auf Rohrreaktoren beschränkt, sondern kann prinzipiell auch bei gerührten Reaktoren zur Anwendung kommen. Ferner ist es möglich, den Katalysator in monolithischer Form einzusetzen.

In der Decarboxylierung von Schritt (VI)(1) können bspw. die folgenden Reaktionsparameter eingehalten werden:
- Temperatur bevorzugt im Bereich von 140 °C bis 240 °C und Druck bevorzugt im Bereich von 1,00 bar_{(abs}.₎ bis 20,0 bar_{(abs.),}
- Temperatur besonders bevorzugt im Bereich von 160 °C bis 220 °C und Druck besonders bevorzugt im Bereich von 1,00 bar_{(abs}.₎ bis 15,0 bar_{(abs.),}
- Temperatur ganz besonders bevorzugt bei einer Temperatur im Bereich von 180 °C bis 200 °C und Druck ganz besonders bevorzugt im Bereich von 4,00 bar_{(abs}.₎ bis 10,0 bar_{(abs).}

Bevorzugt durchläuft der Anilin enthaltende Strom vor seiner Entnahme aus dem Reaktor ein Filter, um zu verhindern, dass Feststoffpartikel (bspw. Katalysatorpartikel) mitgerissen werden.

Die zu decarboxylierende Aminobenzoesäure wird für die Durchführung des Schrittes (VI-1) bevorzugt in Lösung eingesetzt. Als Lösungsmittel eignen sich Wasser oder organische Lösungsmittel wie 1-Dodecanol oder - siehe oben - Anilin.

Schritt (VI)(1) wird bevorzugt kontinuierlich durchgeführt, d. h. dass die zu decarboxylierende Aminobenzoesäure dem Reaktor kontinuierlich zugeführt und das Produkt dem Reaktor kontinuierlich entnommen wird. In einer Variante dieser Verfahrensweise werden auch mindestens Teile des Katalysators im kontinuierlichen Betrieb permanent oder intervallweise gewechselt, um eine Erschöpfung seiner Leistungsfähigkeit zu verhindern. Eine diskontinuierliche Verfahrensführung (sog. "Batch-Fahrweise") ist aber auch möglich. In einer Variante der diskontinuierlichen Fahrweise (sog. "Fed-Batch-Fahrweise") werden die Edukte dem Reaktor so lange kontinuierlich zugeführt, wie es das Reaktorvolumen erlaubt, ohne dass Produkte dem Reaktor entnommen werden. Die Reaktion wird nach Zugabe der maximal möglichen Menge an Edukten unterbrochen und das Produktgemisch dem Reaktor entnommen.

In einer alternativen bevorzugten Ausführungsform ist auch eine Verfahrensführung denkbar, bei welcher zu decarboxylierende Aminobenzoesäure dem Reaktor kontinuierlich zugeführt und das Produkt dem Reaktor kontinuierlich entnommen wird, verbrauchter Katalysator jedoch nicht im kontinuierlichen Betrieb entnommen wird, sondern stattdessen so lange frischer Katalysator (entweder permanent oder intervallweise) zugesetzt wird, bis die durch das vorhandene Reaktorvolumen vorgegebene maximale Katalysatormenge im Reaktor erreicht ist, und dann der Reaktor zwecks Reinigung und Katalysatorwechsel außer Betrieb genommen wird.

In allen Ausführungsformen ist es bevorzugt, Schritt (VI)(1) unter Sauerstoffausschluss durchzuführen. Zur Inertisierung des Reaktors eignen sich inerte Gase wie Stickstoff, Kohlenstoffdioxid oder Edelgase.

Das dem Reaktor von Schritt (VI)(1) entnommene Rohanilin wird vorzugsweise vor seiner weiteren Verwendung aufgereinigt. Diese Aufreinigung kann durch dem Fachmann geläufige Verfahren erfolgen. Bevorzugt umfasst die Aufreinigung mindestens einen Destillationsschritt, welchem eine Wasserabtrennung durch Phasentrennung vorgeschaltet sein kann. Die Aufreinigung kann ebenfalls eine Basenbehandlung zur Entfernung saurer Verunreinigungen vor, während oder nach dem Destillationsschritt umfassen. Geeignete Ausgestaltungen sind bspw. in EP-A-1 845 079, EP-A-1 845 080, EP-A-2 263 997 und EP-A-2 028 176 beschrieben. (Diese Schriften befassen sich mit der Reinigung von Anilin, das durch Hydrierung von Nitrobenzol erhalten wurde; die beschriebenen Reinigungsschritte des Rohanilins sind jedoch auf anders hergestelltes Anilin ebenfalls anwendbar.)

Die weitere Umsetzung so gewonnenen Anilins mit Formaldehyd zu **Di- und Polyaminen der Diphenylmethanreihe (VI)(2)** ist an sich bekannt und kann nach einem beliebigen Verfahren des Standes der Technik durchgeführt werden. Die kontinuierliche oder teilweise diskontinuierliche Herstellung von Di- und Polyaminen der Diphenylmethanreihe aus Anilin und Formaldehyd ist z. B. in EP 1 616 890 A1, US-A-5286760, EP-A-451442 und WO-A-99/40059 offenbart. Die Reaktion erfolgt unter Säurekatalyse. Als saurer Katalysator eignet sich vorzugsweise Salzsäure.

Die weitere Umsetzung der so erhaltenen Di- und Polyamine der Diphenylmethanreihe mit Phosgen zu **Di- und Polyisocyanaten der Diphenylmethanreihe (VI)(3)** ist ebenfalls an sich bekannt und kann nach einem beliebigen Verfahren des Standes der Technik durchgeführt werden. Geeignete Verfahren sind beispielsweise in EP 2 077 150 B1, EP 1 616 857 A1, EP 1 873 142 A1, und EP 0 314 985 B1 beschrieben.

Die Umsetzung des durch Decarboxylierung der erfindungsgemäß erhaltenen Aminobenzoesäure gewonnen Anilins zu **Azoverbindungen, bevorzugt zu Azo-Farbstoffen (VI)(4)** kann nach einem beliebigen Verfahren des Standes der Technik erfolgen. Beispielhaft sei auf die bekannte Herstellung von Anilingelb (para-Aminoazobenzol; CAS 493-5-7) oder Indigo (2,2'-Bis(2,3-dihydro-3-oxomethyliden); CAS 482-89-3) verwiesen (Per Wiklund et al., Current Organic Synthesis, 2006, 3, 379 - 402).

Die Umsetzung der erfindungsgemäß erhaltenen Aminobenzoesäure zu einem **Amid (VI)(5)** kann nach einem beliebigen Verfahren des Standes der Technik erfolgen. Beispielhaft sei das primäre Amin von Anthranilsäure (2-Aminobenzylamid) erwähnt, welches unter anderem als Startmaterial für die Herstellung von Pharmazeutika verwandt wird (Per Wiklund et al., Current Organic Synthesis, 2006, 3, 379 - 402).

Die Umsetzung der erfindungsgemäß erhaltenen Aminobenzoesäure zu einem **leitenden Polymeren wie bevorzugt Polyanthranilsäure (VI)(6)** kann nach einem beliebigen Verfahren des Standes der Technik erfolgen. Ein Beispiel ist in Bhavana Guptaa et al., Polymers Advanced Technologies, 2011, 22, 1982-1988, beschrieben.

Das erfindungsgemäße Verfahren zeichnet sich durch verschiedene Vorteile aus, wie bevorzugt die Folgenden:
- Reduktion der Konzentration an gelöster Aminobenzoesäure im Fermentationsreaktor und dadurch eine Verringerung der Belastung der Mikroorganismen (Aminobenzoesäure ist in höherer Konzentration für viele Mikroorganismen toxisch).
- Das erfindungsgemäße Verfahren ermöglicht es in vorteilhafter Weise, Aminobenzoat durch Einpressen von Kohlenstoffdioxid als Aminobenzoesäure zur Kristallisation zu bringen. Hierdurch wird es ermöglicht, auf die üblicherweise eingesetzten Mineralsäuren, bevorzugt Salzsäure, zu verzichten oder diese allenfalls noch zur Isolierung von Restmengen an Aminobenzoat, wie bevorzugt aus der in Schritt (II)(1) nach der Isolierung des Calcium-Aminobenzoats verbliebenen wässrigen Fermentationslösung, einzusetzen. Außerdem wird das nach Abtrennung der auskristallisierten Aminobenzoeäsure und Entspannung verbleibende Filtrat in den Prozess zurückgeführt (siehe auch FIG. 1) Daher hat die erfindungsgemäße Vorgehensweise neben der Einsparung an Mineralsäuren auch eine erhebliche Reduktion der Belastung des Abwassers mit Salzfracht zur Folge.
- Der erfindungsgemäße Zusatz eines Calcium-Salzes in der Fermentation ermöglicht in vielen Fällen eine erhebliche Einsparung von Base in der Fermentation (in "klassischen" Verfahren wird während der Fermentation häufig Natronlauge zugegeben, um ein Absinken des pH-Werts der Fermentationsbrühe zu vermeiden).

Die Erfindung wird im Folgenden anhand von Beispielen noch näher erläutert.

### Beispiele

*Aus Gründen der sprachlichen Vereinfachung wird im Folgenden in der Regel auch dann von ortho-Aminobenzoesäure gesprochen, wenn diese infolge des herrschenden pH-Werts teilweise oder vollständig als Anion (Aminobenzoat) vorliegt. Hiervon kann abgewichen werden, wenn definierte Aminobenzoat-Verbindungen wie insbesondere ausgefälltes Calcium-ortho-Aminobenzoat oder kommerziell erhältliche Salze bezeichnet werden.*

### Eingesetzte Reagenzien:

oAB-STAMMLÖSUNG: Stammlösung von ortho-Aminobenzoesäure einer Konzentration von 500 g/L, hergestellt durch Auflösen von Natrium-ortho-Aminobenzoat in Wasser bei pH 7,0.

AMMONIUM-STAMMLÖSUNG 1: Stammlösung von Ammoniumcarbonat einer Konzentration von 105 g/L.

AMMONIUM -STAMMLÖSUNG 2: Stammlösung von Ammoniumcarbonat einer Konzentration von 210 g/L.

FERMENTATIONSBRÜHE: Hergestellt durch Fermentation eines ortho-Aminobenzoesäure produzierenden Bakteriums wie in WO 2015/124687 A1, auf S. 35 und S.36 im Abschnitt "General cultivation of *Corynebacterium glutamicum* ATCC13032 based strains", beschrieben; enthält ortho-Aminobenzoesäure in einer Konzentration von 13,0 g/L.

VORKULTURMEDIUM I: Das Medium enthält folgende in vollständig entsalztem Wasser gelöste Komponenten: 16 g/L Soyapeptone, (Duchefa, Lot. No. 021679.01), 10 g/L Hefeextrakt (Gistex LS FERM Batch, AFG2D10), 5 g/L NaCl und 15 g/L Glucose (separat autoklaviert).

VORKULTURMEDIUM II: Das Medium enthält folgende in vollständig entsalztem Wasser gelöste Komponenten: 40 g/L Glukose (separat autoklaviert), 20 g/L (NH₄)₂SO₄, 5 g/L Harnstoff, 42 g/L MOPS-Puffer, 5 g/L Hefeextrakt (Gistex LS FERM Batch AFG2D10), 1 g/L KH₂PO₄, 1 g/L K₂HPO₄, 0,25 g/L M_{g}SO₄·7 H₂O, 0,01 g/L CaCl₂, 2 mg/L Biotin (Zugabe von 1 mL/L einer Biotin-Stammlösung mit 2 g/L Biotin, durch 0,2 µm Filtration sterilisiert) und 1 mL der Spurenelemente-Stammlösung (sterilisiert durch 0,2 µm Filtration)

ANZUCHT-MEDIUM: Das Medium enthält folgende in vollständig entsalztem Wasser gelöste Komponenten: 20 g/L Glukose (separat autoklaviert), 5 g/L (NH₄)₂SO₄ , 4 g/L KH₂PO₄, 4 g/L K₂HPO₄, 2 g/L MgSO₄·7 H₂O, 0,04 g/L CaCl₂·2 H₂O, 5 g/L Hefeextrakt (Gistex LS FERM Batch: AFG2D10), 5 g/L Polypropylenglycol 2000 (Antischaummittel), 2 mg/L Biotin (Zugabe von 1 mL/L einer Biotin-Stammlösung mit 2 g/L Biotin, durch 0,2 µm Filtration sterilisiert) und 10 mL der Spurenelemente-Stammlösung (sterilisiert durch 0,2 µm Filtration).

HAUPTKULTUR-MEDIUM I: Das Medium enthält folgende in vollständig entsalztem Wasser gelöste Komponenten: 40 g/L Glukose (separat autoklaviert), 3,6 g/L (NH₄)₂CO₃, 4 g/L KH₂PO₄, 4 g/L K₂HPO₄, 2 g/L MgSO₄·7 H₂O, 0,04 g/L CaC1₂·2 H₂O, 1 g/L Polypropylenglycol 2000 (Antischaummittel), 2 mg/L Biotin (Zugabe von 1 mL/L einer Biotin-Stammlösung mit 2 g/L Biotin, durch 0,2 µm Filtration sterilisiert) und 10 mL der Spurenelemente-Stammlösung (sterilisiert durch 0,2 µm Filtration).

HAUPTKULTUR-MEDIUM II: Das Medium enthält folgende in vollständig entsalztem Wasser gelöste Komponenten: 40 g/L Glukose (separat autoklaviert), 10 g/L (NH₄)₂CO₃, 3.2 g/L K₂CO₃, 2.25 g/L K₂HPO₄, 2 g/L MgSO₄·7 H₂O, 20 g/L CaCO₃, 1 g/L Polypropylenglycol 2000 (Antischaummittel), 2 mg/L Biotin (Zugabe von 1 mL/L einer Biotin-Stammlösung mit 2 g/L Biotin, durch 0,2 µm Filtration sterilisiert) und 10 mL der Spurenelemente-Stammlösung (sterilisiert durch 0,2 µm Filtration).

SPURENELEMENTE-STAMMLÖSUNG: Wässrige Lösung mit 10 g/L MnSO_{4·}H₂O, 10 g/L FeSO₄·7 H2O, 1 g/L ZnSO₄·7 H₂O, 0,2 g/L CuSO₄·5 H2O, 0,02 g/L NiCl₂·6 H2O. Die Komponenten werden durch Zugabe von HCl bei pH 1 gelöst.

GLUKOSE-TRYPTOPHAN-STAMMLÖSUNG: Wässrige Lösung mit 480 g/L Glukose und 1,6 g/L Tryptophan.

GLUKOSE-STAMMLÖSUNG: 600 g/L Glukoselösung, sterilisiert durch Autoklavieren.

AMMONIAK-BASE: Wässrige Ammoniak-Lösung einer Konzentration, berechnet als NH₃, von 4,5 mol/L.

### Beispiel 1: Prinzipversuch zur Bildung unlöslichen Calcium-ortho-Aminobenzoats in einer Fermentationsbrühe und Auflösen desselben durch Umsalzen mit Ammoniumionen

48,0 g wasserfreien Calciumchlorids wurden zu 1,00 L FERMENTATIONSBRÜHE gegeben. Der pH-Wert wurde durch Zugabe von Salzsäure auf einen Wert von 7,0 eingestellt. Nun wurde diese Mischung durch Zugabe von 200 mL oAB-STAMMLÖSUNG mit 100 g ortho-Aminobenzoesäure (gelöst in Natronlauge bei pH 7,0) versetzt. Es kam augenblicklich zu einem Ausfall von Calcium-ortho-Aminobenzoat. Die in der wässrigen Phase gemessene Konzentration an gelöster ortho-Aminobenzoesäure betrug 18,0 g/L. Der Feststoffanteil des Gemisches wurde abfiltriert und für 48 h bei 80 °C getrocknet. Auf diese Weise wurden 110 g getrockneten Feststoffes erhalten. Davon wurden 20,0 g zu 50,0 mL AMMONIUM-STAMMLÖSUNG 1 gegeben; gerührt und die Konzentration *gelöster* ortho-Aminobenzoesäure bestimmt. Diese betrug 146 g/L.

### Beispiel 2: Prinzipversuch zur Bildung unlöslichen Calcium-ortho-Aminobenzoats in Wasser und Auflösen desselben durch Umsalzen mit Ammoniumionen

48,0 g wasserfreien Calciumchlorids wurden zu 800 mL Wasser gegeben. Der pH-Wert wurde durch Zugabe von Salzsäure auf einen Wert von 7,0 eingestellt. Nun wurde diese Mischung durch Zugabe von 200 mL oAB-STAMMLÖSUNG mit 100 g ortho-Aminobenzoesäure (gelöst in Natronlauge bei pH 7,0) versetzt. Es kam augenblicklich zu einem Ausfall von Calcium-ortho-Aminobenzoat. Die in der wässrigen Phase gemessene Konzentration an gelöster ortho-Aminobenzoesäure betrug 18,0 g/L. Der Feststoffanteil des Gemisches wurde abfiltriert und für 48 h bei 80 °C getrocknet. Auf diese Weise wurden 110 g getrockneten Feststoffes erhalten. Davon wurden 20,0 g zu 25,0 mL AMMONIUM-STAMMLÖSUNG 2 gegeben; gerührt und die Konzentration gelöster ortho-Aminobenoesäure bestimmt. Diese betrug 175 g/L.

### Beispiel 3: Prinzipversuche zur Bestimmung der Löslichkeit an Calcium-ortho-Aminobenzoat in Wasser

3,00 g getrocknetes Calcium-ortho-Aminobenzoat wurden in 100 mL vollständig entsalzten Wassers eingerührt und für 10 min bei Raumtemperatur gerührt. Anschließend wurde in der wässrigen Phase die Konzentration an gelöster ortho-Aminobenoesäure bestimmt. Diese betrug 17,0 g/L.

3,00 g getrocknetes Calcium-ortho-Aminobenzoat wurden in 50 mL vollständig entsalzten Wassers eingerührt und für 10 min bei Raumtemperatur gerührt. Anschließend wurde in der wässrigen Phase die Konzentration an gelöster ortho-Aminobenoesäure bestimmt. Diese betrug 17,5 g/L.

### Beispiele 4 und 5: Fed-batch Fermentation eines ortho-Aminobenzoat produzierenden C.glutamicum Stammes unter Vorlage von Calciumcarbonat

Anzucht einer Vorkultur eines ortho-Aminobenzoat produzierenden *C.glutamicum* Stammes in 25 mL VORKULTURMEDIUM I. Die Kultur wurde in einem 300 mL Erlenmeyerkolben in einem Schüttelinkubator mit einem Schütteldurchmesser von 5 cm bei 30 °C und 200 UpM für 6 Stunden inkubiert.

Im Anschluss wurden 20 mL der Kultur auf 2 x 50 mL VORKULTURMEDIUM II aufgeteilt und in einem Schüttelinkubator mit einem Schütteldurchmesser von 5 cm bei 30 °C und 200 UpM für 5 Stunden inkubiert.

Nach Abschluss der Inkubationszeit für die zweite Vorkultur wurden 40 mL der zweiten Vorkultur in den Anzuchtfermenter überführt. Im Anzuchtfermenter wurde ein Startvolumen von 0,76 L ANZUCHT-MEDIUM vorgelegt, wobei die Menge aller Medienkomponenten, mit Ausnahme von Glukose, für ein Volumen von 1,00 L vorgesehen wurden. Die zugegebene Menge an Glukose wurde so gewählt, dass in einem Volumen von 0,80 L (Vorlagevolumen inkl. Volumen der Vorkultur) eine Konzentration von 40 g/L vorlag. Der Anzuchtfermenter wurde im Fed-Batch Betrieb im Bereich von 5,0 bis 50 g/L Glukose durch Zugabe von GLUKOSE-TRYPTOPHAN STAMMLÖSUNG bei einer Kultivierungstemperatur von 30 °C betrieben. Der pH-Wert wurde im Verlauf der Kultivierung durch die Zugabe von AMMONIAK-BASE konstant gehalten. Der Fermenter wurde mit 0,2 L/min Luft begast, wobei der Gelöstsauerstoff durch Anpassen der Rührerdrehzahl zwischen 200 und 1200 UpM bei 30 % Luftsättigung geregelt wurde. Der Anzucht-Fermenter wurde im Fed-Batch-Betrieb für eine Kultivierungszeit von 24 Stunden betrieben.

Nach Abschluss der Inkubationszeit für den Anzucht-Fermenter wurden 50 mL der Kultur in einen Hauptkultur-Fermenter überführt um eine Start OD₆₀₀ = 20 einzustellen. Es wurden vier Hauptkultur-Fermenter inokuliert, wobei zwei Fermenter mit HAUPTKULTUR-MEDIUM ohne CaCO₃ **(Beispiel 4** - Vergleich) und zwei Fermenter mit zusätzlich 20 g/L CaCO₃ im Medium **(Beispiel 5** - erfindungsgemäßer Schritt *(I))* betrieben wurden. Die Ergebnisse ohne die Zugabe von CaCO₃ sind in **FIG. 2** dargestellt. Die Ergebnisse der Reaktoren, bei denen zusätzlich CaCO₃ zugesetzt wurde, sind in **FIG. 3** dargestellt. Pro Fermenter wurde ein Startvolumen von 0,55 L HAUPTKULTUR-MEDIUM I vorgelegt, wobei die Menge aller Medienkomponenten inkl. CaCO₃, mit Ausnahme von Glukose, für ein Volumen von 1,00 L vorgesehen wurden. Die zugegebene Menge an Glukose wurde so gewählt, dass in einem Volumen von 0,60 L (Vorlagevolumen inkl. Inokulum) eine Konzentration von 40 g/L vorlag. Der Hauptkultur-Fermenter wurde im Fed-Batch Betrieb im Bereich von 5,0 bis 50 g/L Glukose durch Zugabe von GLUKOSE-STAMMLÖSUNG bei einer Kultivierungstemperatur von 30 °C betrieben. Der pH-Wert wurde im Verlauf der Kultivierung durch die Zugabe von AMMONIAK-BASE bei pH = 7,0 konstant gehalten. Der Fermenter wurde mit 0.2 L/min Luft begast, wobei der Gelöstsauerstoff durch Anpassen der Rührerdrehzahl zwischen 200 und 1200 UpM bei 30% Luftsättigung geregelt wurde. Der Hauptkultur-Fermenter wurde im Fed-Batch-Betrieb für eine Kultivierungszeit von 50 Stunden betrieben. Der in Abbildungen FIG.2 und FIG. 3 dargestellte Verlauf von Biotrockenmasse, produzierter ortho-Aminobenzoesäure- (oAB)-Menge und verbrauchter Glukose im Fermentationsverlauf zeigt, dass die Zugabe von CaCO₃ als Feststoff einen positiven Einfluss auf die produzierte Menge an oAB und die umgesetzte Menge an Glukose hat. Das in FIG. 3 dargestellte Trockengewicht enthält den CaCO₃ Feststoff und die getrocknete Biomasse, daher liegt der Startwert an Trockengewicht, im Vergleich zu den Reaktoren ohne CaCO₃-Feststoff in FIG. 2, deutlich höher.

### Beispiel 6: Fed-batch Fermentation eines ortho-Aminobenzoat produzierenden C.glutamicum Stammes unter Vorlage von Calciumcarbonat

Anzucht einer Vorkultur eines ortho-Aminobenzoat produzierenden *C.glutamicum* Stammes in 25 mL VORKULTURMEDIUM I. Die Kultur wurde in einem 300 mL Erlenmeyerkolben in einem Schüttelinkubator mit einem Schütteldurchmesser von 5 cm bei 30 °C und 200 UpM für 6 Stunden inkubiert.

Im Anschluss wurden 20 mL der Kultur auf 2 x 50 mL VORKULTURMEDIUM II aufgeteilt und in einem Schüttelinkubator mit einem Schütteldurchmesser von 5 cm bei 30 °C und 200 UpM für 5 Stunden inkubiert.

Nach Abschluss der Inkubationszeit für die zweite Vorkultur wurden 50 mL der zweiten Vorkultur dirket in einen Hauptkulturfermenter überführt. Es wurden zwei Hauptkulturfermenter betrieben. In den beiden Hauptkulturfermentern wurde jeweils ein Startvolumen von 0,55 L HAUPTKULTUR-MEDIUM II vorgelegt, wobei die Menge aller Medienkomponenten, mit Ausnahme von Glukose, für ein Volumen von 1,00 L vorgesehen wurden. Die zugegebene Menge an Glukose wurde so gewählt, dass in einem Volumen von 0,60 L (Vorlagevolumen inkl. Volumen der Vorkultur) eine Konzentration von 40 g/L vorlag. Die Hauptkulturfermenter wurden im Fed-Batch Betrieb im Bereich von 5,0 bis 50 g/L Glukose durch Zugabe von GLUKOSE- STAMMLÖSUNG bei einer Kultivierungstemperatur von 30 °C betrieben. Die Ergebnisse der Fermentation sind in FIG. 4 und FIG. 5 dargestellt. Um den Start pH-Wert auf einen Wert von unter 8,6 zu reduzierten, wurde in den ersten 15 Stunden der Fermentation der CO₂-Anteil in der Zuluft auf 5 Vol.% eingestellt. Nach einer Fermentationszeit von 15 Stunden wurde die Begasung in beiden Fermentern auf Luft umgestellt und für den weiteren Fermentationsverlauf beibehalten. Durch die Zugabe von AMMONIAK-BASE wurde nach 48 h verhindert, dass der pH Wert unter einen Wert von 6,8 absinkt. Erst nach einer Fermentationszeit von 68 h wurde die Zugabe von AMMONIAK-BASE unterbrochen, um den pH Wert weiter zu reduzieren. Die Fermenter wurden mit einem Volumenstrom von 0,2 L/min eines sauerstoffhaltigen Gasgemisches begast, wobei der Gelöstsauerstoff durch Anpassen der Rührerdrehzahl zwischen 200 und 1200 UpM bei 30 % Luftsättigung geregelt wurde. Der in FIG. 4 dargestellte Verlauf von Biotrockenmasse, produzierter ortho-Aminobenzoesäure- (oAB)-Menge und verbrauchter Glukose im Fermentationsverlauf zeigt, dass die Zugabe von CaCO₃ als Feststoff einen positiven Einfluss auf die produzierte Menge an oAB und die umgesetzte Menge an Glukose hat. Das in FIG. 4 dargestellte Trockengewicht enthält den CaCO₃-Feststoff und die getrocknete Biomasse, daher liegt der Startwert an Trockengewicht, im Vergleich zu den Reaktoren ohne CaCO₃-Feststoff in FIG. 2, deutlich höher. Durch die in FIG. 5 dargestellte pH Reduktion auf einen pH Wert von 6,8 wird das Auflösen von CaCO₃ beschleunigt. Die benötigte Menge an Base im Vergleich zu den Reaktoren ohne CaCO₃-Feststoff in FIG. 2 konnte durch die Pufferwirkung von CaCO₃ um etwa 50 % reduziert werden.

### Beispiel 7: Modell zur Ausfällung von Anthranilsäure aus einer wässrigen Ammonium-anthranilat-Lösung (siehe FIG. 6)

Das Modell wurde im Prozess-Simulationstool AspenPlus geschrieben. Die betrachteten Hauptkomponenten sind Wasser, ortho-Aminobenzoesäure, Ammoniak und CO₂. Das zugrundgelegte thermodynamische Modell berücksichtigt die Gleichgewichtsreaktionen wie bspw. die Formierung von Dihydrogencarbonat, Dissoziationsreaktionen und die Formierung von Salzen bzw. fester Anthranilsäure. Die Gleichgewichts- und Henry-Konstanten stammen aus vorhandenen Datenbanken. Im Modell wird eine einfache Flash-Rechnung durchgeführt, bei der der Dampfanteil gleich 0 gesetzt wird. Berechnet werden somit unter anderem der sich einstellende Druck, der pH-Wert sowie der Anteil an Anthranilsäure, der in fester Form vorliegt. Wie in FIG. 6 dargestellt kann mit dem Modell gezeigt werden, dass mit Hilfe von CO₂ unter einem Druck von 100 bar aus einer wässrigen ortho-Ammoniumanthranilat-Lösung (Massenanteil w_{oAB} = 0.3) 65,8 % der Anthranilsäure auskristallisiert werden kann.

### Beispiel 8: Experiement zur Ausfällung von ortho-Aminobenzoesäure aus einer wässrigen NH₄-ortho-Amninobenzoat-Lösung (siehe FIG. 7)

Wässrige äquimolare NH₄-ortho-Aminobenzoat-Lösungen mit Konzentrationen von 10, 20 und 30 Massen-% an ortho-Aminobenzoesäure wurde in einer temperierten Phasengleichgewichtszelle durch Einpressen von CO₂ unter Druck gesetzt, so dass sich der pH-Wert durch die sich bildende Kohlensäure in der flüssigen Phase verringert. Die definierte CO₂-Zugabe erfolgte durch eine temperierte Spindelpresse. Der pH-Shift hatte einen Ausfall von fester ortho-Aminobenzoesäure zur Folge. Bei mehreren Druckstufen von bis zu 60 bar wurden Proben der flüssigen Phase entnommen und analysiert, um die Konzentration von ortho-Aminobenzoesäure in der flüssigen Phase zu bestimmen. Über eine Massenbilanz wurde im Anschluss der ausgefallene Anteil an ortho-Aminobenzoesäure berechnet. Die Ergebnisse zeigen, dass ein signifikanter Anteil (über 50 % der in der Lösung befindlichen ortho-Aminobenzoesäure) aus der wässrigen Lösung bei einem Druck von bis zu 60 bar kristallisiert werden kann. Durch Entfernen der flüssigen Phase wurde im Anschluss eine Abtrennung des Feststoffs von der flüssigen Phase erzielt. Durch diese Vorgehensweise konnte ortho-Aminobenzoesäure als Feststoff isoliert werden. In FIG. 7 ist der experimentell bestimmte Anteil an ausgefallener ortho-Aminobenzoesäure in Abhängigkeit vom CO₂-Druck dargestellt.

## Patentansprüche

1. Verfahren zur Herstellung von Aminobenzoesäure oder eines Aminobenzoesäure-Folgeprodukts, umfassend die Schritte:
(I) Fermentieren eines Rohstoffes, der wenigstens
• eine fermentierbare Kohlenstoff-haltige Verbindung
und
• eine Stickstoff-haltige Verbindung
umfasst,
in einem Fermentationsreaktor unter Verwendung von Mikroorganismen und eines anorganischen Calcium-Salzes,
sodass ein in einer wässrigen Fermentationslösung suspendiertes Gemisch umfassend nicht gelöste Mikroorganismen und ausgefallenes Calcium-Aminobenzoat erhalten wird;
(II)
(1) Isolieren des in Schritt (I) angefallenen
(1)(i) ausgefallenen Calcium-Aminobenzoats oder
(1)(ii) des Gemisches umfassend nicht gelöste Mikroorganismen und ausgefallenes Calcium-Aminobenzoat
aus der wässrigen Fermentationslösung,
(2) Überführen des im Calcium-Aminobenzoat gebundenen Aminobenzoats in eine wasserlösliche Form unter Bildung eines von Calcium-Aminobenzoat verschiedenen wasserunlöslichen Calcium-Salzes durch Zugabe einer wässrigen Phase, die wasserlösliche Aminobenzoat-Salze bildende Kationen und wasserunlösliche Calcium-Salze bildende Anionen enthält, zu dem isolierten Calcium-Aminobenzoat aus (1)(i) oder zu dem Gemisch umfassend nicht gelöste Mikroorganismen und ausgefallenes Calcium-Aminobenzoat aus (1)(ii), wobei die zugegebene wässrige Phase
Lithium-, Natrium-, Kalium- und/oder Ammonium-Kationen und
Carbonat und/oder Hydrogencarbonat-Anionen,
umfasst,
sodass eine Suspension enthaltend
(2)(i) das ausgefallene wasserunlösliche Calcium-Salz oder
(2)(ii) ein Gemisch umfassend nicht gelöste Mikroorganismen und das wasserunlösliche Calcium-Salz
in einer wässrigen Lösung von Aminobenzoat erhalten wird,
(3) Abtrennen der in Schritt (2) angefallenen wässrigen Lösung von Aminobenzoat vom dem ausgefallenen wasserunlöslichen Calcium-Salz aus (2)(i) oder von dem Gemisch umfassend nicht gelöste Mikroorganismen und das wasserunlösliche Calcium-Salz aus (2)(ii);
(III) Einleiten von Kohlenstoffdioxid bei einem Druck größer oder gleich 1,50 bar_{(abs}.₎ in die in Schritt (II)(3) abgetrennten wässrige Lösung von Aminobenzoat unter Abscheidung von Aminobenzoesäure, sodass eine Suspension enthaltend Aminobenzoesäure in einer wässrigen Lösung gebildet wird;
(IV) Isolierung der in Schritt (III) abgeschiedenen Aminobenzoesäure umfassend eine Druckerniedrigung unter Freisetzung von Kohlenstoffdioxid, wobei eine an Kohlenstoffdioxid abgereicherte und von abgeschiedener Aminobenzoesäure befreiter wässrige Lösung erhalten wird;
(V) Verwenden der in Schritt (IV) erhaltenen, an Kohlenstoffdioxid abgereicherten und von abgeschiedener Aminobenzoesäure befreiten wässrigen Lösung als Bestandteil der in Schritt (II)(2) zugegebenen wässrigen Phase;
(VI) optionale weitere Umsetzung der in Schritt (IV)(1) abgetrennten Aminobenzoesäure zu einem Aminobenzoesäure-Folgeprodukt, wobei Schritt (VI) eine der folgenden Umsetzungen umfasst:
(1) Decarboxylierung der Aminobenzoesäure zu Anilin;
(2) Decarboxylierung der Aminobenzoesäure zu Anilin, gefolgt von säurekatalysierter Reaktion des Anilins mit Formaldehyd unter Bildung von Di- und Polyaminen der Diphenylmethanreihe;
(3) Decarboxylierung der Aminobenzoesäure zu Anilin, gefolgt von säurekatalysierter Reaktion des Anilins mit Formaldehyd unter Bildung von Di- und Polyaminen der Diphenylmethanreihe, gefolgt von Umsetzung mit Phosgen unter Bildung von Di- und Polyisocyanaten der Diphenylmethanreihe;
(4) Decarboxylierung der Aminobenzoesäure zu Anilin, gefolgt von Umsetzung des Anilins zu einer Azoverbindung;
(5) Umsetzung der Aminobenzoesäure zu einem Amid;
(6) Umsetzung der Aminobenzoesäure zu leitenden Polymeren.

2. Verfahren gemäß Anspruch 1, bei welchem das in Schritt (I) eingesetzte anorganische Calcium-Salz ausgewählt ist aus Calciumcarbonat, Calciumhydrogencarbonat, Calciumhydroxid, Calciumoxid oder Mischungen derselben.

3. Verfahren gemäß einem der vorstehenden Ansprüche, bei welchem die Fermentation in Schritt (I) diskontinuierlich in Fermentationszyklen durchgeführt wird.

4. Verfahren gemäß Anspruch 3, bei welchem nach Abschluss eines Fermentationszyklus
(A)
Schritt (II)(1) durchgeführt wird, indem die in Schritt (I) erhaltene wässrige Fermentationslösung unter Zurückhaltung des in dieser suspendierten Gemisches umfassend nicht gelöste Mikroorganismen und ausgefallenes Calcium-Aminobenzoat aus dem Fermentationsreaktor ausgeschleust wird;
Schritt (II)(2) durchgeführt wird, indem die wässrige Phase in den Fermentationsreaktor gegeben wird, sodass im Fermentationsreaktor eine Suspension enthaltend ein Gemisch umfassend nicht gelöste Mikroorganismen und das wasserunlösliche Calcium-Salz in einer wässrigen Lösung von Aminobenzoat erhalten wird;
und
Schritt (II)(3) durchgeführt wird, indem die in Schritt (II)(2) erhaltene wässrige Lösung von Aminobenzoat aus dem Fermentationsreaktor ausgeschleust wird, wobei das Gemisch umfassend nicht gelöste Mikroorganismen und das wasserunlösliche Calcium-Salz zurückgehalten wird und für den nächsten Fermentationszyklus zur Verfügung steht;
oder
(B)
Schritt (II)(1) durchgeführt wird, indem die in Schritt (I) erhaltene wässrige Fermentationslösung gemeinsam mit dem in dieser suspendierten Gemisch umfassend nicht gelöste Mikroorganismen und ausgefallenes Calcium-Aminobenzoat aus dem Fermentationsreaktor ausgeschleust wird und das Gemisch umfassend nicht gelöste Mikroorganismen und ausgefallenes Calcium-Aminobenzoat außerhalb des Fermentationsreaktors von der wässrigen Fermentationslösung getrennt und in den Fermentationsreaktor zurückgeführt wird;
Schritt (II)(2) durchgeführt wird, indem die wässrige Phase in den Fermentationsreaktor gegeben wird, sodass im Fermentationsreaktor eine Suspension enthaltend ein Gemisch umfassend nicht gelöste Mikroorganismen und das wasserunlösliche Calcium-Salz in einer wässrigen Lösung von Aminobenzoat erhalten wird;
und
Schritt (II)(3) durchgeführt wird, indem die in Schritt (II)(2) erhaltene wässrige Lösung von Aminobenzoat aus dem Fermentationsreaktor ausgeschleust wird, wobei das Gemisch umfassend nicht gelöste Mikroorganismen und das wasserunlösliche Calcium-Salz zurückgehalten wird und für den nächsten Fermentationszyklus zur Verfügung steht;
oder
(C)
Schritt (II)(1) durchgeführt wird, indem die in Schritt (I) erhaltene wässrige Fermentationslösung gemeinsam mit dem in dieser suspendierten Gemisch umfassend nicht gelöste Mikroorganismen und ausgefallenes Calcium-Aminobenzoat aus dem Fermentationsreaktor ausgeschleust wird und das Gemisch umfassend nicht gelöste Mikroorganismen und ausgefallenes Calcium-Aminobenzoat außerhalb des Fermentationsreaktors von der wässrigen Fermentationslösung getrennt und in einen vom Fermentationsreaktor verschiedenen Behälter gegeben wird;
Schritt (II)(2) durchgeführt wird, indem die wässrige Phase in diesen Behälter gegeben wird, sodass in diesem Behälter eine Suspension enthaltend ein Gemisch umfassend nicht gelöste Mikroorganismen und das wasserunlösliche Calcium-Salz in einer wässrigen Lösung von Aminobenzoat erhalten wird;
und
nach der Abtrennung der in Schritt (II)(2) erhaltenen wässrigen Lösung von Aminobenzoat von dem Gemisch umfassend nicht gelöste Mikroorganismen und das wasserunlösliche Calcium-Salz in Schritt (II)(3) dieses abgetrennte Gemisch in einem Schritt (II)(4) wieder in den Fermentationsreaktor gegeben wird und für den nächsten Fermentationszyklus zur Verfügung steht.

5. Verfahren gemäß Anspruch 4, bei welchem die Schritte (I) und (II) wiederholt werden, bis in Schritt (IV) die gewünschte Menge an Aminobenzoesäure erhalten wird oder die in Schritt (I) eingesetzten Mikroorganismen erneuert werden müssen.

6. Verfahren gemäß Anspruch 1 oder 2, bei welchem die Fermentation in Schritt (I) kontinuierlich durchgeführt wird.

7. Verfahren gemäß Anspruch 6, bei welchem
(A)
in der wässrigen Fermentationslösung suspendiertes Gemisch umfassend nicht gelöste Mikroorganismen und ausgefallenes Calcium-Aminobenzoat kontinuierlich aus dem Fermentationsreaktor ausgeschleust wird und
Schritt (II)(1) durchgeführt wird, indem nach erfolgter Ausschleusung die unlöslichen Mikroorganismen und ausgefallenes Calcium-Aminobenzoat voneinander und von der wässrigen Fermentationslösung getrennt werden;
Schritt (II)(2) durchgeführt wird, indem die wässrige Phase zu dem so abgetrennten Calcium-Aminobenzoat gegeben wird;
und wobei die in Schritt (II)(1) abgetrennten unlöslichen Mikroorganismen teilweise bis vollständig in den Fermentationsreaktor zurückgeführt werden;
oder bei welchem
(B)
in der wässrigen Fermentationslösung suspendiertes ausgefallenes Calcium-Aminobenzoat unter Zurückhaltung der nicht gelösten Mikroorganismen kontinuierlich aus dem Fermentationsreaktor ausgeschleust wird und
Schritt (II)(1) durchgeführt wird, indem nach erfolgter Ausschleusung ausgefallenes Calcium-Aminobenzoat von der wässrigen Fermentationslösung getrennt wird;
Schritt (II)(2) durchgeführt wird, indem die wässrige Phase zu dem so abgetrennten Calcium-Aminobenzoat gegeben wird;
oder bei welchem
(C)
in der wässrigen Fermentationslösung suspendiertes Gemisch umfassend nicht gelöste Mikroorganismen und ausgefallenes Calcium-Aminobenzoat kontinuierlich aus dem Fermentationsreaktor ausgeschleust wird und
Schritt (II)(1) durchgeführt wird, indem nach erfolgter Ausschleusung das Gemisch umfassend nicht gelöste Mikroorganismen und ausgefallenes Calcium-Aminobenzoat von der wässrigen Fermentationslösung getrennt wird;
Schritt (II)(2) durchgeführt wird, indem die wässrige Phase zu diesem so abgetrennten Gemisch gegeben wird;
und
nach der Abtrennung der in Schritt (II)(2) erhaltenen wässrigen Lösung von Aminobenzoat von dem Gemisch umfassend nicht gelöste Mikroorganismen und das wasserunlösliche Calcium-Salz in Schritt (II)(3) dieses abgetrennte Gemisch in einem Schritt (II)(4) wieder in den Fermentationsreaktor gegeben wird und für die weitere kontinuierliche Fermentation zur Verfügung steht;
oder bei welchem
**(D)**
in der wässrigen Fermentationslösung suspendiertes Gemisch umfassend nicht gelöste Mikroorganismen und ausgefallenes Calcium-Aminobenzoat kontinuierlich aus dem Fermentationsreaktor ausgeschleust wird und
Schritt **(II)**(1) durchgeführt wird, indem nach erfolgter Ausschleusung das Gemisch umfassend nicht gelöste Mikroorganismen und ausgefallenes Calcium-Aminobenzoat von der wässrigen Fermentationslösung getrennt wird;
Schritt **(II)**(2) durchgeführt wird, indem die wässrige Phase zu diesem so abgetrennten Gemisch gegeben wird;
und
nach der Abtrennung der in Schritt **(II)**(2) erhaltenen wässrigen Lösung von Aminobenzoat von dem Gemisch umfassend nicht gelöste Mikroorganismen und das wasserunlösliche Calcium-Salz in Schritt **(II)**(3) dieses abgetrennte Gemisch in einem Schritt **(II)**(4a) in die Bestandteile nicht gelöste Mikroorganismen und wasserunlösliches Calcium-Salz getrennt und in einem Schritt **(II)**(4b) einer der voneinander getrennten Bestandteile wieder in den Fermentationsreaktor gegeben wird und für die weitere kontinuierliche Fermentation zur Verfügung steht.

8. Verfahren gemäß einem der vorstehenden Ansprüche, bei welchem
**(A)**
aus der in Schritt (II)(1) erhaltenen wässrigen Fermentationslösung durch Säurezugabe bis Erreichen eines pH-Werts im Bereich von 3,0 bis < 4,0 Aminobenzoesäure auskristallisiert und die auskristallisierte Aminobenzoesäure isoliert wird, wobei eine an Aminobenzoesäure abgereicherte Mutterlauge verbleibt;
oder bei welchem
**(B)**
die Schritt (II)(1) erhaltene wässrige Fermentationslösung in die Fermentation aus Schritt (I) zurückgeführt wird.

9. Verfahren gemäß einem der vorstehenden Ansprüche, bei welchem Schritt (**IV)** folgende Teilschritte umfasst:
(1) Trennen der abgeschiedenen Aminobenzoesäure und der wässrigen Lösung aus Schritt **(III)** bei einem Druck gleich dem oder größer als der Druck in Schritt **(III),**
(2) Entspannen der in Schritt (1) abgetrennten wässrigen Lösung unter Freisetzung von Kohlenstoffdioxid, wobei eine an Kohlenstoffdioxid abgereicherte wässrige Lösung erhalten wird.

10. Verfahren gemäß einem der vorstehenden Ansprüche, bei welchem das in Schritt (II)(3) abgetrennte wasserunlösliche Calcium-Salz aus (2)(i) oder das Gemisch umfassend nicht gelöste Mikroorganismen und das wasserunlösliche Calcium-Salz aus (2)(ii) in Schritt (I) zurückgeführt wird.

11. Verfahren gemäß einem der vorstehenden Ansprüche, bei welchem das in Schritt (IV) freigesetzte Kohlenstoffdioxid aufgefangen und in Schritt (III) eingesetzt wird.

12. Verfahren gemäß einem der vorstehenden Ansprüche, bei welchem in Schritt (I) Mikroorganismen einer Art umfassend *Escherichia coli, Pseudomonas putida, Corynebacterium glutamicum, Ashbya gossypii, Pichia pastoris, Hansenula polymorpha, Yarrowia lipolytica, Zygosaccharomyces bailii* oder *Saccharomyces cerevisiae* eingesetzt werden.

13. Verfahren gemäß einem der vorstehenden Ansprüche, bei welchem Schritt (I) bei einem pH-Wert von 4,0 oder größer durchgeführt wird.

14. Verfahren gemäß einem der vorstehenden Ansprüche, bei welchem Schritt (II)(2) bei einem pH-Wert von > 7,0 durchgeführt wird.

15. Verfahren gemäß einem der vorstehenden Ansprüche, bei welchem die fermentierbare Kohlenstoff-haltige Verbindung ausgewählt ist aus Stärkehydrolysat, Zuckerrohrsaft, Zuckerrübensaft, Hydrolysaten aus lignocellulosehaltigen Rohstoffen oder Mischungen derselben und bei welchem die Stickstoff-haltige Verbindung ausgewählt ist aus Ammoniakgas, Ammoniakwasser, Ammoniumsalzen, Harnstoff oder Mischungen derselben.

## Claims

1. Process for preparing aminobenzoic acid or an aminobenzoic acid conversion product, comprising the steps of:
(I) fermenting a raw material comprising at least
• a fermentable carbon-containing compound, and
• a nitrogen-containing compound,
in a fermentation reactor using microorganisms and an inorganic calcium salt,
so as to obtain a mixture, suspended in an aqueous fermentation solution, comprising undissolved microorganisms and precipitated calcium aminobenzoate;
(II)
(1) isolating the
(1)(i) precipitated calcium aminobenzoate or
(1)(ii) mixture comprising undissolved microorganisms and precipitated calcium aminobenzoate
obtained in step (I) from the aqueous fermentation solution,
(2) converting the aminobenzoate bound in the calcium aminobenzoate to a water-soluble form to form a water-insoluble calcium salt other than calcium aminobenzoate by adding an aqueous phase containing cations that form water-soluble aminobenzoate salts and anions that form water-insoluble calcium salts to the isolated calcium aminobenzoate from (1)(i) or to the mixture comprising undissolved microorganisms and precipitated calcium aminobenzoate from (1)(ii), wherein the aqueous phase comprises
lithium, sodium, potassium and/or ammonium cations and
carbonate and/or hydrogencarbonate anions,
so as to obtain a suspension comprising
(2)(i) the precipitated water-insoluble calcium salt or
(2)(ii) a mixture comprising undissolved microorganisms and the water-insoluble calcium salt
in an aqueous solution of aminobenzoate,
(3) separating the aqueous solution of aminobenzoate obtained in step (2) from the precipitated water-insoluble calcium salt from (2) (i) or from the mixture comprising undissolved microorganisms and the water-insoluble calcium salt from (2)(ii);
(III) introducing carbon dioxide at a pressure of greater than or equal to 1.50 bar_{(abs}.₎ into the aqueous solution of aminobenzoate separated off in step (II)(3) to separate aminobenzoic acid out, so as to form a suspension containing aminobenzoic acid in an aqueous solution;
(IV) isolating the aminobenzoic acid separated out in step (III), comprising lowering the pressure with release of carbon dioxide to give a carbon dioxide-depleted aqueous solution that has been freed of aminobenzoic acid separated out;
(V) using the aqueous solution obtained in step (IV) that has been depleted of carbon dioxide and freed of aminobenzoic acid separated out as a constituent of the aqueous phase added in step (II) (2);
(VI) optionally further converting the aminobenzoic acid separated off in step (IV) (1) to an aminobenzoic acid conversion product, with step (VI) preferably comprising one of the following conversions:
(1) decarboxylating the aminobenzoic acid to give aniline;
(2) decarboxylating the aminobenzoic acid to give aniline, followed by acid-catalysed reaction of the aniline with formaldehyde to form di- and polyamines of the diphenylmethane series;
(3) decarboxylating the aminobenzoic acid to give aniline, followed by acid-catalysed reaction of the aniline with formaldehyde to form di- and polyamines of the diphenylmethane series, followed by reaction with phosgene to form di- and polyisocyanates of the diphenylmethane series;
(4) decarboxylating the aminobenzoic acid to give aniline, followed by conversion of the aniline to an azo compound;
(5) converting the aminobenzoic acid to an amide;
(6) converting the aminobenzoic acid to conductive polymers.

2. Process according to Claim 1, in which the inorganic calcium salt used in step (I) is selected from calcium carbonate, calcium hydrogencarbonate, calcium hydroxide, calcium oxide and mixtures thereof.

3. Process according to either of the preceding claims, in which the fermentation in step (I) is performed batchwise in fermentation cycles.

4. Process according to Claim 3, in which, on conclusion of a fermentation cycle,
**(A)**
step (II) (1) is conducted by discharging the aqueous fermentation solution obtained in step (I) from the fermentation reactor while retaining the mixture comprising undissolved microorganisms and precipitated calcium aminobenzoate suspended therein;
step (II) (2) is conducted by introducing the aqueous phase into the fermentation reactor so as to obtain a suspension containing a mixture comprising undissolved microorganisms and the water-insoluble calcium salt in an aqueous solution of aminobenzoate in the fermentation reactor;
and
step (II) (3) is conducted by discharging the aqueous solution of aminobenzoate obtained in step (II)(2) from the fermentation reactor, while retaining the mixture comprising undissolved microorganisms and the water-insoluble calcium salt and making it available for the next fermentation cycle;
or
**(B)**
step (II) (1) is conducted by discharging the aqueous fermentation solution obtained in step (I) from the fermentation reactor together with the mixture comprising undissolved microorganisms and precipitated calcium aminobenzoate suspended therein and separating the mixture comprising undissolved microorganisms and precipitated calcium aminobenzoate from the aqueous fermentation solution outside the fermentation reactor and recycling it into the fermentation reactor;
step (II) (2) is conducted by introducing the aqueous phase into the fermentation reactor so as to obtain a suspension containing a mixture comprising undissolved microorganisms and the water-insoluble calcium salt in an aqueous solution of aminobenzoate in the fermentation reactor;
and
step (II) (3) is conducted by discharging the aqueous solution of aminobenzoate obtained in step (II) (2) from the fermentation reactor, while retaining the mixture comprising undissolved microorganisms and the water-insoluble calcium salt and making it available for the next fermentation cycle;
or
(C)
step (II)(1) is conducted by discharging the aqueous fermentation solution obtained in step (I) from the fermentation reactor together with the mixture comprising undissolved microorganisms and precipitated calcium aminobenzoate suspended therein and separating the mixture comprising undissolved microorganisms and precipitated calcium aminobenzoate from the aqueous fermentation solution outside the fermentation reactor and introducing it into a vessel other than the fermentation reactor;
step (II)(2) is conducted by introducing the aqueous phase into this vessel so as to obtain a suspension containing a mixture comprising undissolved microorganisms and the water-insoluble calcium salt in an aqueous solution of aminobenzoate in this vessel;
and
after the separation of the aqueous solution of aminobenzoate obtained in step (II)(2) from the mixture comprising undissolved microorganisms and the water-insoluble calcium salt in step (II)(3), this mixture separated off is introduced back into the fermentation reactor in a step (II)(4) and made available for the next fermentation cycle.

5. Process according to Claim 4, in which steps (I) and (II) are repeated until the desired amount of aminobenzoic acid is obtained in step (IV) or the microorganisms used in step (I) have to be replaced.

6. Process according to Claim 1 or 2, in which the fermentation in step (I) is performed continuously.

7. Process according to Claim 6, in which
(A)
mixture suspended in the aqueous fermentation solution and comprising undissolved microorganisms and precipitated calcium aminobenzoate is discharged continuously from the fermentation reactor and
after discharging, step (II) (1) is conducted by separating the insoluble microorganisms and precipitated calcium aminobenzoate from one another and from the aqueous fermentation solution;
step (11)(2) is conducted by adding the aqueous phase to the calcium aminobenzoate thus separated off;
and wherein the insoluble microorganisms separated off in step (II)(1) are recycled partly to completely into the fermentation reactor;
or in which
(B)
precipitated calcium aminobenzoate suspended in the aqueous fermentation solution is discharged continuously from the fermentation reactor while retaining the undissolved microorganisms and
after discharging, step (II) (1) is conducted by separating precipitated calcium aminobenzoate from the aqueous fermentation solution;
step (II)(2) is conducted by adding the aqueous phase to the calcium aminobenzoate thus separated off;
or in which
(C)
mixture suspended in the aqueous fermentation solution and comprising undissolved microorganisms and precipitated calcium aminobenzoate is discharged continuously from the fermentation reactor and
after discharging, step (II)(1) is conducted by separating the mixture comprising undissolved microorganisms and precipitated calcium aminobenzoate from the aqueous fermentation solution;
step (II)(2) is conducted by adding the aqueous phase to this mixture thus separated off;
and
after the separation of the aqueous solution of aminobenzoate obtained in step (II)(2) from the mixture comprising undissolved microorganisms and the water-insoluble calcium salt in step (II)(3), this mixture separated off is introduced back into the fermentation reactor in a step (II)(4) and made available for the further continuous fermentation;
or in which
(D)
mixture suspended in the aqueous fermentation solution and comprising undissolved microorganisms and precipitated calcium aminobenzoate is discharged continuously from the fermentation reactor and
after discharging, step (II)(1) is conducted by separating the mixture comprising undissolved microorganisms and precipitated calcium aminobenzoate from the aqueous fermentation solution;
step (II)(2) is conducted by adding the aqueous phase to this mixture thus separated off;
and
after the separation of the aqueous solution of aminobenzoate obtained in step (II)(2) from the mixture comprising undissolved microorganisms and the water-insoluble calcium salt in step (II)(3), this mixture separated off is separated in a step (II)(4a) into the constituents of undissolved microorganisms and water-insoluble calcium salt, and, in a step (II) (4b), one of the constituents separated from one another is returned to the fermentation reactor and made available for the further continuous fermentation.

8. Process according to any of the preceding claims, in which
**(A)**
aminobenzoic acid is crystallized out of the aqueous fermentation solution obtained in step (II)(1) by adding acid until attainment of a pH in the range from 3.0 to < 4.0 and the crystallized aminobenzoic acid is isolated, leaving an aminobenzoic aciddepleted mother liquor;
or in which
(B)
the aqueous fermentation solution obtained in step (II)(1) is recycled into the fermentation from step (I).

9. Process according to any of the preceding claims, in which step (IV) comprises the following partial steps:
(1) separating the aminobenzoic acid separated out and the aqueous solution from step (III) at a pressure equal to or greater than the pressure in step (III),
(2) decompressing the aqueous solution separated off in step (1) to release carbon dioxide, giving a carbon dioxide-depleted aqueous solution.

10. Process according to any of the preceding claims, in which the water-insoluble calcium salt from (2)(i) that has been separated off in step (II)(3) or the mixture comprising undissolved microorganisms and the water-insoluble calcium salt from (2)(ii) is recycled into step (I).

11. Process according to any of the preceding claims, in which the carbon dioxide released in step (IV) is collected and used in step (III).

12. Process according to any of the preceding claims, in which microorganisms of a type comprising *Escherichia coli, Pseudomonas putida, Corynebacterium glutamicum, Ashbya gossypii, Pichia pastoris, Hansenula polymorpha, Yarrowia lipolytica, Zygosaccharomyces bailii* or *Saccharomyces cerevisiae* are used in step (I).

13. Process according to any of the preceding claims, in which step (I) is conducted at a pH of 4.0 or greater.

14. Process according to any of the preceding claims, in which step (II) (2) is conducted at a pH of > 7.0.

15. Process according to any of the preceding claims, in which the fermentable carbon-containing compound is selected from starch hydrolysate, sugarcane juice, sugarbeet juice, hydrolysates of lignocellulose-containing raw materials or mixtures thereof, and in which the nitrogen-containing compound is selected from gaseous ammonia, aqueous ammonia, ammonium salts, urea or mixtures thereof.

## Revendications

1. Procédé de préparation d'acide aminobenzoïque ou d'un dérivé de l'acide aminobenzoïque, comprenant les étapes :
(I) fermentation d'une matière première qui comprend au moins
• un composé carboné fermentable et
• un composé azoté,
dans un réacteur de fermentation par utilisation de microorganismes et d'un sel de calcium inorganique,
de façon à obtenir un mélange en suspension dans une solution aqueuse de fermentation, comprenant des microorganismes non dissous et de l'aminobenzoate de calcium précipité ;
(II)
(1) isolement, à partir de la solution aqueuse de fermentation,
(1)(i) de l'aminobenzoate de calcium précipité obtenu ou
(1)(ii) du mélange obtenu, comprenant des microorganismes non dissous et de l'aminobenzoate de calcium précipité,
(2) conversion de l'aminobenzoate lié dans l'aminobenzoate de calcium en une forme soluble dans l'eau avec formation d'un sel de calcium insoluble dans l'eau, différent de l'aminobenzoate de calcium, par addition d'une phase aqueuse qui contient des cations formant des sels aminobenzoates solubles dans l'eau et des sels de calcium insolubles dans l'eau, à l'aminobenzoate de calcium isolé de (1)(i) ou au mélange comprenant des microorganismes non dissous et de l'aminobenzoate de calcium précipité de (1)(ii), la phase aqueuse ajoutée comprenant
des cations lithium, sodium, potassium et/ou ammonium et
des anions carbonate et/ou hydrogénocarbonate,
de façon à obtenir une suspension contenant
(2)(i) le sel de calcium insoluble dans l'eau précipité ou
(2)(ii) un mélange comprenant des microorganismes non dissous et le sel de calcium insoluble dans l'eau, dans une solution aqueuse d'aminobenzoate,
(3) séparation de la solution aqueuse d'aminobenzoate obtenue dans l'étape (2) du sel de calcium insoluble dans l'eau précipité de (2)(i) ou du mélange comprenant des microorganismes non dissous et le sel de calcium insoluble dans l'eau de (2)(ii) ;
(III) introduction de dioxyde de carbone sous une pression supérieure ou égale à 1,50 bar_{(abs.)} dans la solution aqueuse séparée dans l'étape (II)(3) d'aminobenzoate avec dépôt d'acide aminobenzoïque, de façon à former une suspension contenant de l'acide aminobenzoïque en solution aqueuse ;
(IV) isolement de l'acide aminobenzoïque déposé dans l'étape (III), comprenant une diminution de la pression avec libération de dioxyde de carbone, avec obtention d'une solution aqueuse appauvrie en dioxyde de carbone et débarrassée de l'acide aminobenzoïque déposé ;
(V) utilisation de la solution aqueuse obtenue dans l'étape (IV), appauvrie en dioxyde de carbone et débarrassée de l'acide aminobenzoïque déposé, comme constituant de la phase aqueuse ajoutée dans l'étape (II)(2) ;
(VI) réaction plus poussée éventuelle de l'acide aminobenzoïque séparé dans l'étape (IV)(1) pour obtenir un dérivé de l'acide aminobenzoïque, l'étape (VI) comprenant l'une des réactions suivantes :
(1) décarboxylation de l'acide aminobenzoïque en aniline ;
(2) décarboxylation de l'acide aminobenzoïque en aniline, suivie d'une réaction, catalysée par un catalyseur acide, de l'aniline avec du formaldéhyde avec formation de di- et de polyamines de la série du diphénylméthane ;
(3) décarboxylation de l'acide aminobenzoïque en aniline, suivie d'une réaction, catalysée par un catalyseur acide, de l'aniline avec du formaldéhyde avec formation de di- et de polyamines de la série du diphénylméthane, suivie d'une réaction avec du phosgène avec formation de di- et de polyisocyanates de la série du diphénylméthane ;
(4) décarboxylation de l'acide aminobenzoïque en aniline, suivie de la conversion de l'aniline en un composé azoïque ;
(5) conversion de l'acide aminobenzoïque en un amide ;
(6) conversion de l'acide aminobenzoïque en des polymères conducteurs.

2. Procédé selon la revendication 1, dans lequel le sel de calcium inorganique utilisé dans l'étape (I) est choisi parmi le carbonate de calcium, l'hydrogénocarbonate de calcium, l'hydroxyde de calcium, l'oxyde de calcium ou les mélanges de ceux-ci.

3. Procédé selon l'une des revendications précédentes, dans lequel la fermentation est mise en œuvre dans l'étape (I) d'une manière discontinue dans des cycles de fermentation.

4. Procédé selon la revendication 3, dans lequel, après la fin d'un cycle de fermentation,
(A)
on met en œuvre l'étape (II)(1), en évacuant du réacteur de fermentation la solution aqueuse de fermentation obtenue dans l'étape (I), avec retenue du mélange, en suspension dans celle-ci, comprenant des microorganismes non dissous et de l'aminobenzoate de calcium précipité ;
on met en œuvre l'étape (II)(2) en introduisant la phase aqueuse dans le réacteur de fermentation, de façon à obtenir dans le réacteur de fermentation une suspension contenant un mélange comprenant des microorganismes non dissous et le sel de calcium insoluble dans l'eau dans une solution aqueuse d'aminobenzoate ;
et
on met en œuvre l'étape (II)(3) en évacuant du réacteur de fermentation la solution aqueuse d'aminobenzoate obtenue dans l'étape (II)(2), le mélange comprenant des microorganismes non dissous et le sel de calcium insoluble dans l'eau étant retenu et étant disponible pour le cycle de fermentation suivant ;
ou
(B)
on met en œuvre l'étape (II)(1) en évacuant du réacteur de fermentation la solution aqueuse de fermentation obtenue dans l'étape (I), en même temps que le mélange en suspension dans cette dernière, comprenant des microorganismes non dissous et de l'aminobenzoate de calcium précipité, et on sépare de la solution aqueuse de fermentation, à l'extérieur du réacteur de fermentation, le mélange comprenant des microorganismes non dissous et de l'aminobenzoate de calcium précipité, et on le renvoie dans le réacteur de fermentation ;
on met en œuvre l'étape (II)(2) en introduisant la phase aqueuse dans le réacteur de fermentation, de façon à obtenir dans le réacteur de fermentation une suspension contenant un mélange comprenant des microorganismes non dissous et le sel de calcium insoluble dans l'eau dans une solution aqueuse d'aminobenzoate ;
et
on met en œuvre l'étape (II)(3) en évacuant du réacteur de fermentation la solution aqueuse d'aminobenzoate obtenue dans l'étape (II)(2), le mélange comprenant des microorganismes non dissous et le sel de calcium insoluble dans l'eau étant retenu et étant disponible pour le cycle de fermentation suivant ;
ou
(C)
on met en œuvre l'étape (II)(1) en évacuant du réacteur de fermentation la solution aqueuse de fermentation obtenue dans l'étape (I) en même temps que le mélange en suspension dans cette dernière, comprenant des microorganismes non dissous et de l'aminobenzoate de calcium précipité, et on sépare de la solution aqueuse de fermentation, à l'extérieur du réacteur de fermentation, le mélange comprenant des microorganismes non dissous et de l'aminobenzoate de calcium précipité, et on l'introduit dans un récipient différent du réacteur de fermentation ;
on met en œuvre l'étape (II)(2) en introduisant la phase aqueuse dans ce récipient, de façon à obtenir dans ce récipient une suspension contenant un mélange comprenant des microorganismes non dissous et le sel de calcium insoluble dans l'eau dans une solution aqueuse ;
et
après la séparation de la solution aqueuse d'aminobenzoate obtenue dans l'étape (II)(2) du mélange comprenant des microorganismes non dissous et le sel de calcium insoluble dans l'eau de l'étape (II)(3), ce mélange séparé est, dans une étape (II)(4), réintroduit dans le réacteur de fermentation et est disponible pour le cycle de fermentation suivant.

5. Procédé selon la revendication 4, dans lequel les étapes (I) et (II) sont répétées jusqu'à ce que, dans l'étape (IV), on obtienne la quantité souhaitée d'acide aminobenzoïque, ou bien qu'il faille renouveler les microorganismes utilisés dans l'étape (I).

6. Procédé selon la revendication 1 ou 2, dans lequel la fermentation de l'étape (I) est mise en œuvre en continu.

7. Procédé selon la revendication 6, dans lequel
(A)
on évacue en continu du réacteur de fermentation le mélange en suspension dans la solution aqueuse de fermentation des microorganismes non dissous et de l'aminobenzoate de calcium précipité, et
on met en œuvre l'étape (II)(1) en séparant les uns de l'autre et de la solution aqueuse de fermentation, après la fin de l'évacuation, les microorganismes insolubles et l'aminobenzoate de calcium précipité ;
on met en œuvre l'étape (II)(2) en ajoutant la phase aqueuse au benzoate de calcium ainsi séparé ;
et dans lequel les microorganismes insolubles séparés dans l'étape (II)(1) sont, partiellement à complètement, renvoyés dans le réacteur de fermentation ;
ou dans lequel
(B)
l'aminobenzoate de calcium précipité, en suspension dans la solution aqueuse de fermentation, est évacué en continu du réacteur de fermentation avec retenue des microorganismes non dissous et
l'étape (II)(1) est mise en œuvre en séparant de la solution aqueuse de fermentation l'aminobenzoate de calcium précipité après la fin de l'évacuation ;
on met en œuvre l'étape (II)(2) en ajoutant la phase aqueuse à l'aminobenzoate de calcium ainsi séparé ;
ou dans lequel
(C)
le mélange en suspension dans la solution aqueuse de fermentation, comprenant des microorganismes non dissous et de l'aminobenzoate de calcium précipité, est évacué en continu du réacteur de fermentation et
on met en œuvre l'étape (II)(1) en séparant de la solution aqueuse de fermentation, après la fin de l'évacuation, le mélange comprenant des microorganismes non dissous et de l'aminobenzoate de calcium précipité ; on met en œuvre l'étape (II)(2) en ajoutant la phase aqueuse à ce mélange ainsi séparé ;
et
après séparation de la solution aqueuse d'aminobenzoate obtenue dans l'étape (II)(2) du mélange comprenant des microorganismes non dissous et le sel de calcium insoluble dans l'eau de l'étape (II)(3), ce mélange séparé est, dans une étape (II)(4), réintroduit dans le réacteur de fermentation et est disponible pour la fermentation continue supplémentaire ;
ou dans lequel
(D)
on évacue en continu du réacteur de fermentation le mélange en suspension dans la solution aqueuse de fermentation, comprenant des microorganismes non dissous et de l'aminobenzoate de calcium précipité et on met en œuvre l'étape (II)(1) en séparant de la solution aqueuse de fermentation, après la fin de l'évacuation, le mélange comprenant des microorganismes non dissous et de l'aminobenzoate de calcium précipité ;
on met en œuvre l'étape (II)(2) en ajoutant la phase aqueuse à ce mélange ainsi séparé ;
et
après séparation de la solution aqueuse d'aminobenzoate obtenue dans l'étape (II)(2) du mélange comprenant des microorganismes non dissous et le sel de calcium insoluble dans l'eau de l'étape (II)(3), ce mélange séparé est, dans une étape (II) (4a), séparé en les constituants microorganismes non dissous et sel de calcium insoluble dans l'eau et, dans une étape (II) (4b), l'un des constituants séparés les uns des autres est réintroduit dans le réacteur de fermentation et est disponible pour la fermentation continue supplémentaire.

8. Procédé selon l'une des revendications précédentes, dans lequel
(A)
on sépare par cristallisation l'acide aminobenzoïque de la solution aqueuse de fermentation obtenue dans l'étape (II)(1) par addition d'un acide jusqu'à atteindre un pH dans la plage de 3,0 à < 4,0, et on isole l'acide aminobenzoïque séparé par cristallisation, en obtenant un résidu de lessive-mère appauvrie en acide benzoïque ; ou dans lequel
(B)
on renvoie dans la fermentation de l'étape (I) la solution aqueuse de fermentation obtenue dans l'étape (II)(1).

9. Procédé selon l'une des revendications précédentes, dans lequel l'étape (IV) comprend les étapes partielles suivantes :
(1) séparation de l'acide aminobenzoïque déposé et de la solution aqueuse de l'étape (III) sous une pression supérieure ou égale à la pression de l'étape (III),
(2) détente de la solution aqueuse séparée dans l'étape (I) avec libération de dioxyde de carbone, avec obtention d'une solution aqueuse appauvrie en dioxyde de carbone.

10. Procédé selon l'une des revendications précédentes, dans lequel le sel de calcium insoluble dans l'eau de (2)(i) séparé dans l'étape (II)(3) ou le mélange comprenant des microorganismes non dissous et le sel de calcium insoluble dans l'eau de (2)(ii), est renvoyé dans l'étape (I).

11. Procédé selon l'une des revendications précédentes, dans lequel le dioxyde de carbone libéré dans l'étape (IV) est recueilli et utilisé dans l'étape (III) .

12. Procédé selon l'une des revendications précédentes, dans lequel dans l'étape (I) on utilise des microorganismes d'une espèce comprenant *Escherichia coli, Pseudomonas putida, Corynebacterium glutamicum, Ashbya gossypii, Pichia pastoris, Hansenula polymorpha, Yarrowia lipolytica, Zygosaccharomyces bailii* ou *Saccharomyces cerevisiae.*

13. Procédé selon l'une des revendications précédentes, dans lequel l'étape (I) est mise en œuvre à un pH de 4,0 ou plus.

14. Procédé selon l'une des revendications précédentes, dans lequel l'étape (II)(2) est mise en œuvre à un pH > 7,0.

15. Procédé selon l'une des revendications précédentes, dans lequel le composé carboné fermentable est choisi parmi l'hydrolysat d'amidon, le jus de canne à sucre, le jus de betterave à sucre, les hydrolysats de matières premières lignocellulosiques ou des mélanges de ces derniers, et dans lequel le composé azoté est choisi parmi l'ammoniac gazeux, l'ammoniaque, les sels d'ammonium, l'urée ou les mélanges de ceux-ci.
